# EUROPEAN PATENT SPECIFICATION

(11) **EP 1 856 247 B1**
(45) Date of publication and mention of the grant of the patent: **04.05.2016**
(21) Application number: 06718839.1
(22) Date of filing: 19.01.2006
(51) Int. Cl.: A61K 31/00, A61P 35/00, A61K 45/06, A61K 31/4706, A61K 31/675

(54) **REGULATION OF AUTOPHAGY AND CELL SURVIVAL**
REGULIERUNG VON AUTOPHAGIE UND ZELLÜBERLEBEN
RÉGULATION DE L'AUTOPHAGIE ET DE LA SURVIE CELLULAIRE

(30) Priority: 19.01.2005 US 645419 P
(43) Date of publication of application: 21.11.2007
(73) Proprietor: The Trustees of The University of Pennsylvania, Philadelphia, Pennsylvania 19104-6283 (US)
(72) Inventor: THOMPSON, Craig, B., Merion, PA 19066 (US); LUM, Julian, Philadelphia, PA 19128 (US); BAUER, Daniel, Philadelphia, PA 19103 (US)
(74) Representative: Leonard, Thomas Charles
(86) International application number: PCT/US2006/001831
(87) International publication number: WO 2006/078774

(56) References cited:
- WO-A1-2004/024676
- WO-A2-2004/100885
- FR-A1- 2 857 012
- BOSSA R ET AL: "Effect of various anti-tumor drugs on energetic processes" PHARMACOLOGICAL RESEARCH COMMUNICATIONS, vol. 8, no. 4, 1 August 1976 (1976-08-01), pages 369-377, XP024888398 ITALIAN PHARMACOLOGICAL SOCIETY, IT ISSN: 0031-6989 DOI: 10.1016/0031-6989(76)90037-0 [retrieved on 1976-08-01]
- DEVRIM GOZUACIK AND ADI KIMCHI: "Autophagy as a cell death and tumor suppressor mechanism" ONCOGENE, vol. 23, no. 16, 12 April 2004 (2004-04-12), pages 2891-2906, XP008121265 NATURE PUBLISHING GROUP, GB ISSN: 0950-9232 DOI: 10.1038/SJ.ONC.1207521
- NG S S W ET AL: "EFFECTS OF THE EPIDERMAL GROWTH FACTOR RECEPTOR INHIBITOR OSI-774, TARCEVA, ON DOWNSTREAM SIGNALING PATHWAYS AND APOPTOSIS IN HUMAN PANCREATIC ADENOCARCINOMA" MOLECULAR CANCER THERAPEUTICS, vol. 1, no. 10, 1 August 2002 (2002-08-01), pages 777-783, XP009056210 AMERICAN ASSOCIATION OF CANCER RESEARCH, US ISSN: 1535-7163
- KNOUZY BURHAN ET AL: "Ifosfamide metabolite chloroacetaldehyde inhibits cell proliferation and glucose metabolism without decreasing cellular ATP content in human breast cancer cells MCF-7" JOURNAL OF APPLIED TOXICOLOGY, vol. 30, no. 3, April 2010 (2010-04), pages 204-211, XP8127707 ISSN: 0260-437X
- MAZUREK SYBILLE ET AL: "Effect of extracellular AMP on cell proliferation and metabolism of breast cancer cell lines with high and low glycolytic rates" JOURNAL OF BIOLOGICAL CHEMISTRY, vol. 272, no. 8, 1997, pages 4941-4952, XP8127673 ISSN: 0021-9258
- MIKURIYA ET AL.: "Expression of glycolytic enzymes is increased in pancreatic cancerous tissues..." INT. J. OF ONCOLOGY, vol. 30, 2007, pages 849-855, XP002603609
- HULKOWER KEREN I ET AL: "Stimulus dependence of non-steroidal antiinflammatory drug potency in a cellular assay of prostaglandin H synthase-2" EUROPEAN JOURNAL OF PHARMACOLOGY, vol. 331, no. 1, 1997, pages 79-85, XP8127719 ISSN: 0014-2999
- CSUKA M ET AL: "TREATMENT OF INTRACTABLE RHEUMATOID ARTHRITIS WITH COMBINED CYCLOPHOSPHAMIDE AZATHIOPRINE AND HYDROXYCHLOROQUINE A FOLLOW-UP STUDY" JOURNAL OF THE AMERICAN MEDICAL ASSOCIATION, vol. 255, no. 17, 1986, pages 2315-2319, XP8127720 ISSN: 0098-7484
- YAMADA K ET AL: "Chemoimmunotherapy of acute myelogenous leukemia in adults with BCG cell-wall skeleton" GANN MONOGRAPHS ON CANCER RESEARCH, vol. 21, 1978, pages 199-209, XP8127765 JP ISSN: 0072-0151
- FERNANDEZ YAMILET ET AL: "Effect of systemic lupus erythematosus (SLE) treatment drugs on GI-101A breast tumor cell growth" LIFE SCIENCES, vol. 67, no. 5, 23 June 2000 (2000-06-23), pages 567-575, XP8127722 ISSN: 0024-3205
- GANG-JUN DUA, ZHI-HUI SONGA, HAI-HONG LINA, XIAO-FENG HANA, SHUO ZHANGA AND YI-MING YANGB: "Luteolin as a glycolysis inhibitor offers superior efficacy and lesser toxicity of doxorubicin in breast cancer cells" BIOCHEMICAL AND BIOPHYSICAL RESEARCH COMMUNICATIONS, vol. 372, no. 3, 2008, pages 492-502, XP002604189
- ISIDORO A, ET AL.: "Breast carcinomas fulfill the warburg hypothesis and provide metabolic markers of cancer prognosis" CARCINOGENESIS, vol. 26, no. 12, 20 July 2005 (2005-07-20), pages 2095-2104, XP002604220
- GOZUACIK D.: 'Autophagy as a cell death and tumor suppressor mechanism' ONCOGENE vol. 23, 2004, pages 2891 - 2906, XP008121265
- TELBISZ AGNES ET AL: "Intracellular protein degradation and autophagy in isolated pancreatic acini of the rat", CELL BIOCHEMISTRY AND FUNCTION, vol. 18, no. 1, March 2000 (2000-03), pages 29-40, ISSN: 0263-6484
- H PELICANO ET AL: 'Glycolysis inhibition for anticancer treatment' ONCOGENE vol. 25, no. 34, 07 August 2006, pages 4633 - 4646, XP055009657 DOI: 10.1038/sj.onc.1209597 ISSN: 0950-9232
- LAUREN J AKERS ET AL: "Targeting glycolysis in leukemia: A novel inhibitor 3-BrOP in combination with rapamycin", LEUKEMIA RESEARCH, NEW YORK,NY, US, vol. 35, no. 6, 29 December 2010 (2010-12-29), pages 814-820, XP028383564, ISSN: 0145-2126, DOI: 10.1016/J.LEUKRES.2010.12.028 [retrieved on 2011-01-03]
- ZHANG ET AL: 'antitumour activity of chloroquine...' ASIAN PAC J CANCER PREV vol. 16, no. 9, pages 3907 - 3912
- RANGWALA ET AL: 'phase 1 trial of hydroxychloroquine...' AUTOPHAGY vol. 10, no. 8, pages 1369 - 1379
- GOLDEN ET AL: 'Chloroquine enhances tenozolomide...' NEUROSURG FOCUS vol. 37, no. 6, 2014, page E12
- FUKUDA ET AL: 'the antimalarial chloroquine...' GYNECOL ONCOL vol. 137, no. 3, 2015, pages 538 - 545

## Description

### FIELD OF THE INVENTION

The present invention relates to regulation of autophagy and cell survival and compositions for use in methods of treating of cancer.

### BACKGROUND OF THE INVENTION

This application claims priority to U.S. Provisional Application Number 60/645,419, filed January 19, 2005.

Animal cells depend on extrinsic factors to provide signals for growth and proliferation. When these signals are lost, both growth and division cease, and programmed cell death is initiated through the intrinsic mitochondrial pathway. An additional consequence of growth factor limitation is a rapid decline in the surface expression of nutrient transporters including the major glucose transporter GLUT1, the LDL receptor, amino acid transporters and receptors for iron uptake. This decrease in nutrient transporter expression has been proposed to perturb mitochondrial physiology resulting in the induction of apoptotic cell death. However, an alternative explanation is that the decline in surface expression of nutrient transporters simply reflects a secondary response to the decreased metabolic demand on the cell following the cessation of growth and the withdrawal from the cell cycle. In this model, perturbations in mitochondrial physiology result from the activities of apoptotic regulatory factors.

In yeast, which lack the central components of the mitochondrial apoptotic pathway, nutrient deprivation results in compromised bioenergetics and activation of a cell survival response termed macroautophagy. During macroautophagy regions of the cytosol become sequestered in double membrane vesicles known as autophagosomes. Upon fusion with the vacuole, the contents of autophagosomes are degraded and the resulting degradation products are then either reutilized to maintain basal macromolecular synthesis or oxidized in the mitochondria to maintain bioenergetics. Yeast can survive for several weeks in the absence of extracellular nutrients through macroautophagy. A role for autophagy in organismal survival during starvation has been observed in plants, worms, flies and mice. In addition, autophagy has been reported to initiate cell death in response to intracellular damage caused by hypoxia, chemotherapeutic agents, virus infection, or toxins. This may contribute to disease pathology as macroautophagy has been observed in a variety of neurodegenerative diseases. Macroautophagy initiated as a response to intracellular damage may provide a multicellular organism with a mechanism to eliminate damaged cells even if the ability of the cells to respond by apoptosis becomes impaired.

Recently, Bax and Bak have been demonstrated to be required for cells to initiate apoptosis through the intrinsic mitochondrial pathway. Cells from Bax^{-/-}Bak^{-/-} animals fail to undergo apoptosis in response to serum deprivation, loss of attachment and growth factor withdrawal. Thus, Bax and Bak are essential and redundant regulators of apoptosis and extracellular signals are no longer necessary to suppress mitochondrial initiation of apoptosis in Bax^{-/-}Bak^{-/-} cells.

One characteristic of cancer cells is they often do not undergo apoptosis. Chemotherapy is used to eliminate these non-apoptotic cells. However, recurrence of cancer subsequent to chemotherapy is observed in some patients. Such recurrence is often attributed to micrometastases which are not eliminated by chemotherapy. There remains a need to improve and develop new cancer treatments to reduce the incidence of recurrence. There remains a need to identify new cancer treatment methods, and new compositions and compounds useful to treat cancer.

Cell death that occurs in degenerative diseases is often observed to be associated with autophagy. Several drugs have been developed with the goal of inhibiting degeneration and cell death associated with degenerative diseases. There remains a need to improve and develop new treatments for degenerative diseases. There remains a need to identify new treatment methods, and new compositions and compounds useful to treat degenerative diseases.

### SUMMARY OF THE INVENTION

The subject matter for which protection is sought is as set out in the claims.

The present invention provides a pharmaceutical composition or kit comprising:
a first anti-cancer composition which is a compound that converts glycolysis dependent cancer cells to cells incapable of glycolysis and is an alkylating agent and
a second anti-cancer composition which is an autophagy inhibitor selected from the group consisting of: chloroquine, hydroxychloroquine and shRNA against the autophagy gene ATG5;
the pharmaceutical composition or kit for use in the treatment of a glycolysis dependent cancer in a human, wherein the treatment comprises administering to the individual said first anti-cancer composition and said second anti-cancer composition.

The present invention also provides the use of a combination of:
an anti-cancer composition, wherein said anti-cancer composition is a compound that converts glycolysis dependent cancer cells to cells incapable of glycolysis and is an alkylating agent, and
an autophagy inhibitor selected from the group consisting of: chloroquine, hydroxychloroquine, and shRNA against the autophagy gene ATG5; in the manufacture of a medicament for treating a glycolysis dependent cancer

The present invention further provides a pharmaceutical composition comprising an autophagy inhibitor selected from the group consisting of: chloroquine, hydroxychloroquine, and a shRNA against the autophagy gene ATG5 for use in the treatment of a glycolysis dependent cancer, wherein the autophagy inhibitor is for simultaneous, separate or sequential administration with an anti-cancer composition that converts glycolysis dependent cancer cells to cells incapable of glycolysis, and wherein the anti-cancer composition is an alkylating agent.

The present invention still further provides an autophagy inhibitor for use in the treatment of a glycolysis dependent cancer, wherein the autophagy inhibitor is selected from the group consisting of: chloroquine, hydroxychloroquine, and a shRNA against the autophagy gene ATG5 and is for simultaneous, separate or sequential administration with an anti-cancer composition that converts glycolysis dependent cancer cells to cells incapable of glycolysis, and wherein the anti-cancer composition is an alkylating agent.

### BRIEF DESCRIPTION OF THE FIGURES

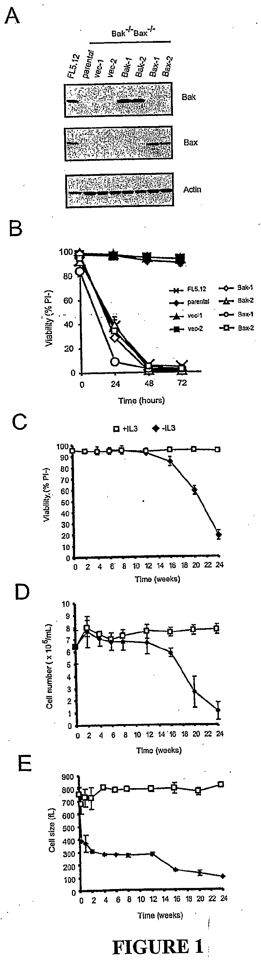

Figure 1 shows that Bax^{-/-}Bak^{-/-} cells undergo atrophy and maintain prolonged survival following withdrawal of growth factor. In Panel (A), two independent clones of Bax^{-/-}Bak^{-/-} IL-3 dependent cells (parental) stably transfected with either Bax, Bak or empty vector (vec) were generated and expression levels were assessed by Western blot. The IL-3-dependent Bax^{+/+}Bak^{+/+} cell line FL5.12 is shown for comparison. In Panel (B), kinetics of cell death in Bax- or Bak-reconstituted cells following IL-3 withdrawal were evaluated. Viability was measured by propidium iodide exclusion. Data are averages of 3 experiments ± standard deviation (S.D.). In Panel (C), cell viability of Bax^{-/-}Bak^{-/-} cells in the presence or absence of IL-3 was evaluated. Cells were washed and cultured in the presence (open squares) or absence (closed diamonds) of IL-3. At the indicated time points, cells were collected and viability was assessed. Cells grown in the presence of IL-3 were passaged every 2-3 days to restore a cell concentration of 7.5 x 10⁵ cells/mL. The medium in IL-3 deprived cultures was replaced with an identical volume of fresh complete medium without IL-3 every 10 days. Data are averages of 3 independent experiments ± S.D. Panel (D) shows cell numbers of cultures that were grown in the presence or absence of IL-3 and were cultured as in Panel (C). Data are averages of 3 independent experiments ± S.D. Panel (E) shows cell size of cultures that were grown in the presence or absence of IL-3 and were cultured as in Panel (C). Data are averages of 3 independent experiments ± S.D.

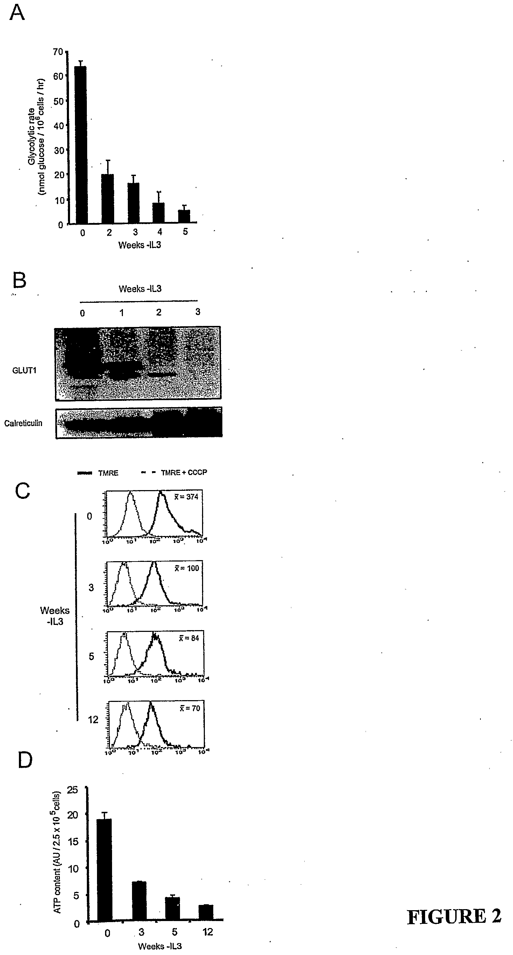

Figure 2 shows the metabolic effects of IL-3 withdrawal on Bax^{-/-}Bak^{-/-} cells. In Panel (A) glycolytic rate of cells grown in the absence of IL-3 was measured by the conversion of 5-³H-glucose to ³H₂O at the indicated time points. The data presented at week 0 represents values of control cells growing in IL-3 throughout the time course of the experiment. Data are averages of three experiments ± S.D. Panel (B) shows results from Western blot analysis of GLUT 1 expression in cells cultured in the absence of IL-3. The GLUT 1 expression at week 0 is representative of GLUT 1 expression of cells grown in IL-3. In Panel (C), mitochondrial membrane potential as measured by TMRE staining in cells grown without IL-3 (solid histogram) at the indicated time point. Baseline TMRE was determined by using cells treated with the uncoupler CCCP (dotted histogram). The numbers in the top right corner indicate the average mean fluorescence intensity of 3 independent experiments. The week 0 time point indicates the mean fluorescence intensity of cells growing in IL-3 and is representative of the values obtained for such cells over the time course of the experiment. In Panel (D), ATP levels in cells grown without IL-3 were evaluated and are expressed as arbitrary units (AU). ATP levels for IL-3 grown cells did not decline significantly over the time course of the experiment (data not shown). Data are averages of 3 independent experiments ± S.D.

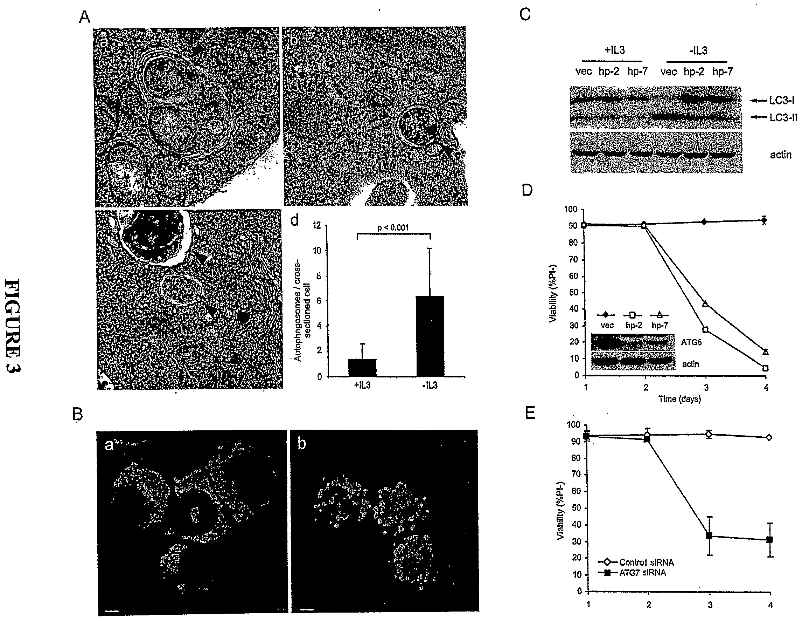

Figure 3 shows that growth factor withdrawal induced autophagosome formation is required for survival. Panel (A) shows results from electron microscopy of cells grown in the absence of IL-3 for 48 hours (subpanel a-c) showing the presence of autophagosomes. Arrowheads depict representative autophagosomes quantitated in subpanel (d). Scale bar, 100 nm. In subpanel (d) quantitation of the number of autophagosomes per cross-sectioned cell cultured in the presence or absence of IL-3 for 48 hours was done. Error bar represents ± S.D. Statistical significance determined by Student's t-test. Panel (B) shows results from immunofluoresence assays with anti-LC3 antibody on cells grown in the presence (a) or absence (b) of IL-3 for 48 hours. Scale bar, 5 µm. Panel (C) shows data from immunoblot analysis of LC3-I processing into LC3-II in cells transfected with control or two independent shRNA constructs against *ATG5* (hp-2 and hp-7) followed by culture in the presence (data not shown) or absence of IL-3 for 48 hours. Actin was used as a loading control. Panel (D) shows data from a time course of cell viability following IL-3 withdrawal in cells with inactivation of *ATG5.* Data are averages of 3 experiments ± S.D. Western blot analysis of *ATG5* protein expression in cells transfected with vector control, hp-2 or hp-7 shRNA is shown as a representative experiment. Actin was used as loading control. Panel (E) shows data from a time course of cell viability following IL-3 withdrawal in cells transfected with FITC tagged siRNA for *ATG7* (Yu et al., 2004) or a control siRNA. Cells which had incorporated the siRNA for *ATG7* or control were purified by FACS sorting based on FITC positive cells and viability was assessed at the indicated time points. Data are averages of 3 experiments ± S.D.

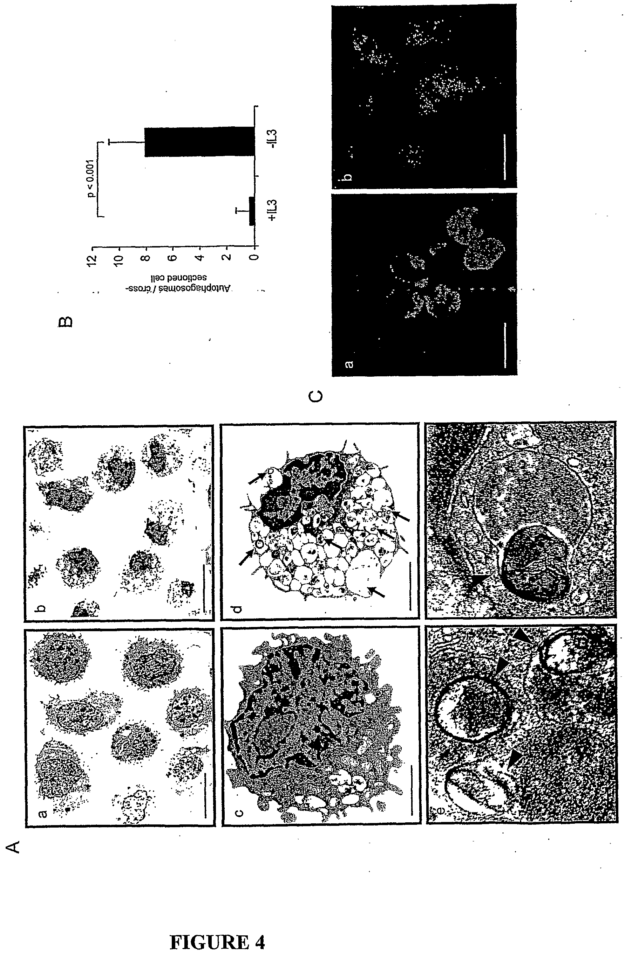

Figure 4 shows persistent autophagy in long term growth factor withdrawn cells. Panel (A) shows electron microscopy data of cells grown in the presence (subpanel a) or absence (subpanel b) of IL-3 for 6 weeks. Scale bar, 8.5 µm. Magnification image of a cell grown in the presence (subpanel c) or absence (subpanel d) of IL-3 showing autophagosomes (arrows). Scale bar, 2.3 µm. Higher magnification of cells grown in the absence of IL-3 (subpanel e and subpanel f). Arrowheads depict autophagosomes in cells containing recognizable cellular material (subpanel e) or a late autophagosome fusing with a lysosome (subpanel f). Arrowheads depict representative autophagosomes quantitated in Panel (B). Scale bar, 100 µm. Panel (B) shows quantitation of the number of autophagosomes per cross-sectioned cells cultured in the presence or absence of IL-3 for 6 weeks. Error bar represents ± S.D. Statistical significance was determined by Student's t-test. Panel (C) shows data from immunofluorescence of cells grown in the presence (a) or absence (b) of IL-3 for 6 weeks probed with anti-LC3 antibodies to detect autophagosomes. Scale bar, 12 µm.

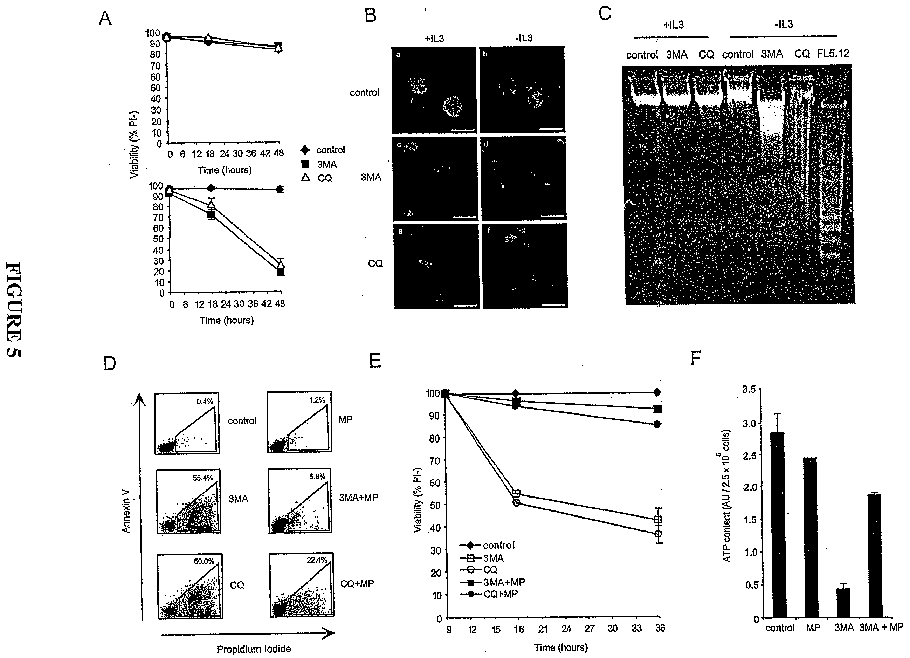

Figure 5 shows cell death following inhibition of autophagy is reversed by methylpyruvate. Panel (A) shows viability of cells grown in the presence (top panel) or absence (bottom panel) of IL-3 for 6 weeks treated with 5 mM 3-MA (closed squares) or 10 µM CQ (open triangles). PBS was used as a vehicle control (closed diamonds). Data represent averages of 3 experiments ± S.D. Panel (B) shows data from immunofluorescence staining of LC3 in cells grown in the presence (subpanel a) or absence (subpanel b) of IL-3 for 6 weeks. Cells grown in the presence or absence of IL-3 were treated for 18 hours with 5 mM 3-MA (subpanel c and subpanel d) or 10 µM CQ (subpanel e and subpanel f) followed by LC3 staining. PBS was used as a vehicle control. Scale bar, 10 µm. Panel (C) shows data from a DNA fragmentation assay that was performed on Bax^{-/-}Bak^{-/-} cells grown in the presence or absence of IL-3 for 6 weeks and treated for 36 hours with 5 mM 3-MA, 10 µM CQ or PBS as a vehicle control. IL-3-dependent Bax^{+/+}Bak^{+/+} FL5.12 cells grown in the absence of IL-3 for 36 hours were used as a positive control for DNA laddering. Panel (D) shows viability of cells grown in the absence of IL-3 for 6 weeks after 18 hours of treatment with 5 mM 3-MA or 10 µM CQ in the presence or absence of 10 mM methylpyruvate (MP). Panel (E) shows viability of cells deprived of IL-3 for 6 weeks that were treated with 5 mM 3-MA or 10 µM CQ (open symbols) in the presence (closed squares and circles) or absence of 10 mM MP. Control cells were left untreated (closed diamonds). Data represent averages of 3 experiments ± S.D. Panel (F) shows ATP levels of cells grown in the presence or absence of IL-3. Cells withdrawn from IL-3 for 6 weeks were treated with 10 mM MP or 5 mM 3-MA alone or together for 8 hours. ATP levels expressed as arbitrary units (AU). Data represent average of 3 independent experiments ± S.D.

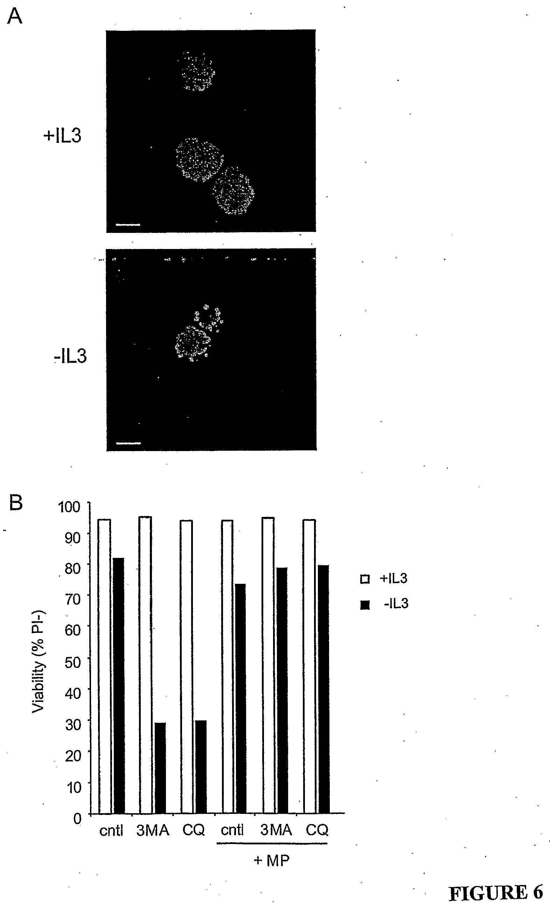

Figure 6 shows cell survival in primary bone marrow Bax^{-/-}Bak^{-/-} cells is controlled by macroautophagy and growth factor availability. Panel (A) shows immunofluorescence staining with anti-LC3 antibodies of cells cultured in the presence or absence of IL-3 for 14 days. Scale bar, 5 µm. Panel (B) shows data from Bax^{-/-}Bak^{-/-} bone marrow cells were cultured in the presence (open bars) or absence (solid bars) of IL-3 for 14 days. On day 14, cells were treated with 5 mM 3-MA or 10 µM CQ in the presence or absence of 10 mM MP. Cell viability was assessed by propidium iodide exclusion 36 hours later.

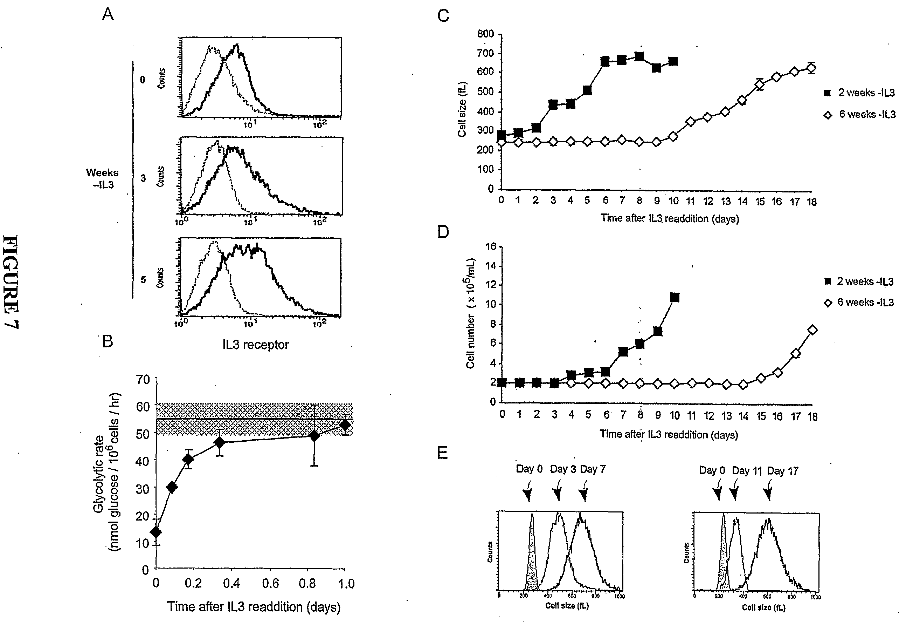

Figure 7 shows that IL-3 restimulates glycolysis and growth/proliferation in growth factor-deprived cells. Panel (A) shows cell surface staining of IL-3 receptor alpha chain. Dotted histogram represents isotype control and solid histogram represents IL-3 receptor expression. Representative of 3 independent experiments. Panel (B) shows the glycolytic rate of cells following readdition of IL-3. IL-3 was readded to cells that were cultured in the absence of IL-3 for 4 weeks and collected at the indicated time points for measurement of glycolytic rate. Solid line indicates average glycolytic rate of cells grown in the presence of IL-3 over the time course of the experiment. The hatched area represents ± 1 S.D. Representative of 3 independent experiments. Panels (C and D) show cell size and cell number of cultures cultured without IL-3 for 2 (closed squares) or 6 (open triangles) weeks followed by readdition of IL-3. Data represent average of 3 experiments ± S.D. Panel (E) shows cell size recovery following IL-3 readdition is dependent on the duration of deprivation. Histogram of mean cell size (fL) in cells restimulated with IL-3 for the indicated number of days following 2 (left panel) or 6 (right panel) weeks of growth factor withdrawal.

Figures 8A-8E refer to the effects of chloroquine or ATG5 knockdown on the growth of MYC/p53ER^{TAM} lymphomas. Figure 8A shows that Chloroquine (CQ) impairs lymphoma cell growth in vitro. Lymphoma cells were harvested from a primary Myc/p53ER^{TAM} tumor, passaged in culture and the medium was changed daily. Cell number was measured daily by Coulter counter. Results are mean ± S.D. of triplicate samples from a representative experiment. *p<0.05, **p<0.005. Figure 8B shows a CQ dose-response curve. Cells were cultured in medium that was changed daily and cell number was measured on day 4. The IC₅₀ value was determined to be 3 µM. Results are mean ± SD of triplicate samples from a representative experiment. Figure 8C shows results from a Western blot analysis of ATG5 knockdown in Myc/p53ER^{TAM} lymphoma cells transduced with the pKD (vector) or pKD/shATGS (shATG5). Actin was used as a loading control. Figure 8D shows data from Knockdown of ATG5 and CQ treatment. Cell number was measured daily by Coulter counter and the medium was changed daily. Mean ± S.D. of triplicate samples from a representative experiment. p<0.005 for CQ treated shVector compared to untreated shVector cells. Figure 8E shows that CQ impairs tumor growth. Cells from a primary MYC/p53ER^{TAM} tumor were harvested and passaged in vivo in syngeneic C57BL/6X129F1 mice. Cells were injected subcutaneously into the flanks of mice. When tumors reached a volume of>1000 mm³, mice were assigned to daily PBS IP, daily CQ 60 mg/kg/day IP, or daily CQ 50 mg/kg/day IP. Results shown are mean ± SD daily tumor volumes and are representative of multiple experiments (*p<0.005, †p<0.05).

Figure 9A-9D refer to the effects of p53 activation ± chloroquine on the regression of MYC/p53ER^{TAM} lymphomas Figure 9A data show Chloroquine (CQ) enhances p53-induced tumor regression and delays tumor recurrence. Myc/p53ER^{TAM} cells were injected subcutaneously into the flanks of 18 C57BL/6X129F1 mice. Once tumors reached>1500 mm³, mice were assigned to daily treatment (↓) with tamoxifen (TAM) 1 mg/day intraperitoneally (IP) + saline (TAM/PBS) or TAM 1 mg/day IP + CQ 60 mg/kg/day IP (TAM/CQ). Results shown are daily tumor volumes (mean ± SD) for each group from a representative experiment (*p<0.05,**p<0.005). Figure 9B show CQ-induced cell death after p53 activation. Electron micrographs (original magnification 4000X) of lymphoma tissues collected before TAM treatment and after 48 hours of TAM/PBS or TAM/CQ. Figure 9C show data of a time course of changes in autophagosomes during tumor regression. Electron micrographs (original magnification 10,000X) of lymphoma tissues collected at 96 hours of PBS or CQ treatment alone, and at 8 hours, 24 hours, and 48 hours after initiation of TAM treatment; double-membrane vesicles (↑). Figure 9D show ultrastructural changes induced by p53 activation with or without CQ treatment at 24 hours. Electron micrographs (original magnification 0,000X).

Figure 10 refers to effects of p53 activation ± chloroquine on autophagosome accumulation Quantification of tumor cells with increased autophagosomes. Electron microscopy was performed on Myc/p53ER^{TAM} lymphomas at the indicated time points and treatments. The percentage of cells (mean ± SD) per high-powered field (4000x) with intact nuclei and >3 double membrane vesicles determined for >3 high powered fields per tumor until>100 total cells were counted as described in experimental procedures (*p<0.005). N.S.: not significant.

Figures 11A and 11B refer to effects of p53 activation ± chloroquine on apoptosis. Figure 11A show data from TUNEL staining that was performed on tissue obtained from treated tumors at the indicated time points as described in experimental procedures. Representative images were obtained by fluorescent microscopy. Red fluorescence indicates TUNEL positive cells. Blue fluorescence indicates nuclear DAPI staining. Figure 11B show quantification of TUNEL positive tumor cells. The percent TUNEL positive cells per high-powered field was determined as described in experimental procedures. Results are mean ± SD.

Figures 12A-12C refer to in vitro effects of chloroquine and ATG5 knockdown after p53 activation. Figure 12A show data from GFP-LC3 fluorescence assays. A bulk population of primary Myc/p53ER^{TAM}lymphoma cells with stable expression of the GFP-LC3 fusion protein were treated with ± 4hydroxytamoxifen (hTAM) 250 nM and ± chloroquine (CQ) 5pM. Cell culture medium was changed daily. Cells were fixed and imaged using fluorescence microscopy at 48 hours. Figure 12B show activation of p53ER^{TAM} + ATG5 knockdown. Daily viable cell number by trypan blue exclusion of yc/p53ER^{TAM}/vector cells and Myc/p53ER^{TAM}/shATG5 cells after daily exposure to hTAM 250 nM. Figure 12C show activation of p53 + CQ, activation of p53 + ATG5 knockdown + CQ. Viable cell number of Myc/p53ER^{TAM}/ vector treated with hTAM 250 nM + CQ 5 µM compared to untreated Myc/p53ER^{TAM}/vector cells. Exposure of Myc1p53ER^{TAM}/shATG5 cells to hTAM 10 mM + CQ 5 pM. For (12B)-(12C) viable cell number was counted daily until the original cell number plated before initiation of hTAM treatment was surpassed (---). Results are mean ± SD of triplicate samples from a representative experiment.

Figure 13 refers to effects of cyclophosphamide ± chloroquine in MYC/p53ERTAM lymphomas: Cells from a primary tumor were harvested and passaged in vivo in syngeneic C57BL/6X129F1 mice. MYC/p53ER^{TAM} cells were injected subcutaneously into the flanks of mice. Once tumors had reached>1700 mm³ mice were matched for tumor size and were treated with cyclophosphamide 50 mg/kg IP once followed by either daily PBS IP or daily chloroquine 60 mg/kg/day IP for a total of thirteen days. Daily tumor volumes are shown for individual mice.

### DETAILED DESCRIPTION OF PREFERRED EMBODIMENTS

### Treatment of Cancer

As used herein, "glycolysis dependent cancer" is meant to refer to cancer that is characterized by cancer cells that rely on glucose metabolism for essentially all of their energy needs excluding energy that may be obtained by autophagy. Cancer cells of glycolysis dependent cancer may be capable of some level of non-glycolytic metabolism but such level does not prevent the cancer cells from undergoing cell death by apoptosis or autophagy in the absence of a glucose energy source.

As used herein, "incapable of glycolysis" is meant to refer to the ability to perform essentially no glucose metabolism. Cancer cells incapable of glycolysis may be able to perform some level of glycolysis but such level does not prevent the cancer cell from undergoing cell death by apoptosis or autophagy in the absence of an alternative means of energy.

As used herein, "autophagy inhibitor" is meant to refer to composition which decreases the level of autophagy in a cell undergoing autophagy in its presence compared to the level of autophagy in a cell undergoing autophagy in its absence. The autophagy inhibitors used in the invention are chloroquine, hydroxychloroquine, and shRNA against the autophagy gene ATG5.

One aspect of the present invention relates to compositions for use in methods of treating individuals who have cancer glycolysis dependent cancer. Cancer which relies upon glycolysis can be identified by several well know techniques, particularly Positron Emission Tomography using ¹⁸fluoro-2-deoxyglucose (FDG-PETscan). When chemotherapy interferes with the ability of such cancers to metabolize glucose, the cancer cells must rely upon autophagy as a survival mechanism until they can resume glycolysis. The methods of the invention employ the use of a combination of compounds to eliminate cancer cells. One or more compounds are administered to the individual to interfere with the cancer cell's ability to metabolize glucose, thereby inducing the cell to rely upon autophagy for survival. In combination with the compound or compounds that bring about the cessation of glycolysis, one or more autophagy inhibitors are administered to the individual to eliminate the cancer's ability to use autophagy as a survival mechanism and the cancer cell dies.

Many cancers rely upon glucose metabolism. The tissue origin of the cancer is not itself indicative of whether or not the cancer that an individual is dependent upon glycolysis. Cancer originating from any of many tissue types frequently is dependent upon aerobic glycolysis.

There are numerous methods of determining whether or not a cancer is dependent upon glycolysis. Samples of tumors can be excised and examined *in vitro* by any one of several well known assays to determine if the cells are dependent on glycolysis. Such methods can determine whether or not the cells utilize aerobic or anaerobic glycolysis. FDG-PETscan technology uses high levels of glucose uptake as a marker for detection. The cancer cells that take up the detectable glucose derivative ¹⁸fluoro-2-deoxyglucose can be located on a computer image of the patient's anatomy. Those cancers which can be detected by FDG-PETscan technology have a high likelihood of being dependent on glycolysis.

PET methodologies are set forth in Czernin, J. 2002 Acta Medica Austriaca 29:162-170. Many cancers are characterized by a high rate of glycolysis wherein the cancer has cells which exhibit a higher rate of glycolysis than that of the tissue surrounding it. Such cancer cells take up above-average quantities of glucose from the environment. Cancer characterized by a high rate of glycolysis can be identified using PET imaging technology, preferably with ¹⁸fluoro-deoxyglucose. The positive detection of a tumor using such a test indicates that the cancer is characterized by glycolysis.

Certain chemotherapies interfere with the cancer cells ability to perform glycolysis. This prevention of the cancer from utilizing glucose can be observed using FDG-PETscan. Essentially, cancer cells which rely on glucose metabolism are PETscan positive and become PETscan negative when treated with certain chemotherapeutics. Because they cannot process glucose, cancer cells treated with such compounds will not preferably take up ¹⁸fluoro-2-deoxyglucose and thus will no longer be detectable by PETScan. Thus, cancer cells which are converted from PETscan positive to PETscan negative, i.e. from relying on glycolysis to not being able to use glucose, undergo autophagy as a survival mechanism. These cancer cells which rely upon autophagy for cell survival and which later are believed to be a source of cancer recurrence can be induced to die by inhibiting autophagy and denying the cell the ability to use autophagy as a survival mechanism.

Many classes of chemotherapeutics and many known compounds converted PETscan positive cancer to PETscan negative. The ability to determine whether a compound can do so is routine.

Similarly, many known compounds are known to inhibit autophagy. One aspect of the present disclosure provides an assay to identify compounds that can inhibit autophagy.

According to the invention, individuals who have cancer that is reliant on glucose metabolism are treated by the use of the combination of an alkylating agent that results in cessation of glucose metabolism and an autophagy inhibitor selected from the group consisting of chloroquine, hydroxychloroquine and shRNA against the autophagy gene ATG5. This combination of interventions leaves the cell unable to utilize outside sources of nutrients as well as energy sources provided by the cells own components. Thus, the cell is deprived of all energy sources and dies.

An individual who has cancer is first identified as having a cancer which is glycolysis dependent. In some preferred embodiments, a PETscan, preferably using ¹⁸fluoro-2-deoxyglucose, is performed prior to administration of anti-cancer compounds. In some embodiments, a sample of tumor is removed from the patient and tested *in vitro* for glycolysis dependency. In some embodiments, the methods include performing PETscan or glycolysis assays to identify the individual as having a cancer that is glycolysis dependent. In some embodiments, the methods include reviewing PETscan or assay data to identify the individual as having a cancer that is glycolysis dependent. In some embodiments, the methods include performing PETscan or glycolysis assays or reviewing PETscan or assay data after administration of a compound intended to render the cancer incapable of glycolysis to confirm cessation of glycolysis in the previously PETscan positive or assay positive cancer.

The combination of pharmaceutical compounds may be administered to the patient in any particular order so long as the autophagy inhibitor is present following conversion of the cancer cells from being capable of performing glycolysis to being unable to perform glycolysis. In some embodiments, the alkylating agent that renders the glycolysis dependent cancer cells incapable of glycolysis is administered prior to the administration of the one or more compounds that inhibit autophagy. In some embodiments, the alkylating agent that renders the glycolysis dependent cancer cells incapable of glycolysis is administered simultaneously with the administration of the one or more compounds that inhibit autophagy. In some embodiments, the alkylating agent that renders the glycolysis dependent cancer cells incapable of glycolysis is administered subsequent to the administration of the one or more compounds that inhibit autophagy.

In some parts of the present disclosure, the anti-cancer compound that converts a cancer cell dependent on glycolysis into a cancer cell whose capability for glycolysis is so impaired such that it is essentially incapable of glycolysis is a compound from a class of compounds selected from the group consisting of: Alkylating Agents; Nitrosoureas; Antitumor Antibiotics; Corticosteroid Hormones; Anti-estrogens; Aromatase Inhibitors; Progestins; Anti-androgens; LHRH agonists; Antibody therapies; and other anti-cancer therapies. Examples of Alkylating Agents include busulfan, cisplatin, carboplatin, chlorambucil, cyclophosphamide, ifosfamide, dacarbazine (DTIC), mechlorethamine (nitrogen mustard), and melphalan. Examples of Nitrosoureas include carmustine (BCNU) and lomustine (CCNU). Examples of Antitumor Antibiotics include dactinomycin, daunorubicin, doxorubicin (Adriamycin), idarubicin, and mitoxantrone. Examples of Corticosteroid Hormones include prednisone and dexamethasone. Examples of anti-estrogens include tamoxifen and fulvestrant. Examples of aromatase inhibitors include anastrozole and letrozole. An example of a Progestin is megestrol acetate. Examples of anti-androgens include bicalutamide, flutamide. Examples of LHRH agonists include leuprolide and goserelin. Examples of antibody therapies include Herceptin and Avastin. Examples of other anti-cancer compounds include L-asparaginase and tretinoin. In some embodiments, combinations or two or more anti-cancer compounds may be used. The present invention uses alkylating agents.

In some parts of the present disclosure, the autophagy inhibitor is selected from the group consisting of: chloroquine, 3-methyladenine, hydroxychloroquine (Plaquenil™), bafilomycin A1, 5-amino-4-imidazole carboxamide riboside (AICAR), okadaic acid, autophagy-suppressive algal toxins which inhibit protein phosphatases of type 2A or type 1, analogues of cAMP, and drugs which elevate cAMP levels, adenosine, N6-mercaptopurine riboside, wortmannin, and vinblastine. In addition, antisense or siRNA that inhibits expression of proteins essential for autophagy, such as for example ATG5, may also be used. The present invention uses chloroquine, hydroxychloroquine or shRNA against the autophagy gene ATG5.

In some embodiments, the individual undergoes surgery and or radiation treatment as part of their therapy.

In any embodiments, anti-cancer compounds and autophagy inhibitors which are used may be used in many cases as presently formulated. Alternatively the pharmaceutical composition or compositions may be formulated by one having ordinary skill in the art for delivery in the therapeutically effective dose for a chosen mode of administration as described in Example 2.

In some embodiments, pharmaceutical kits are provided which contain a first anti-cancer compound and a second anti-cancer compound and instructions for their use in combination. In some embodiments, pharmaceutical kits are provided which contain a first anti-cancer compound and a second anti-cancer compound which are packaged in a separate containers. In some embodiments, pharmaceutical kits are provided which contain a first anti-cancer compound and a second anti-cancer compound which are packaged in a unitary container which has separate compartments or sections such as for example a blister pack.

The pharmaceutical compositions and kits comprise a first anti-cancer compound and a second anti-cancer compound in doses which are therapeutically effective when used in combination.

According to some embodiments of the invention, the individual has been diagnosed as having a type of cancer set forth in Example 3.

### Treatment of Degenerative Diseases (Not part of the invention)

Degenerative diseases and conditions include neurodegenerative or CNS degenerative diseases such as: Alzheimer's Disease; Lewy Body Diseases; Multi-infarct Dementia; Pick's Diseasel; Huntington's Disease; Parkinson's Disease; Amyotrophic Lateral Sclerosis; Creutzfeldt-Jakob Disease; Frontal lobe degeneration (FLD), also called frontotemporal dementia or non-specific frontal lobe dementia; Corticobasal degeneration; Multiple system atrophy and Progressive supranuclear palsy.

One aspect of the present disclosure relates to methods of treating individuals who have a degenerative disease by administering a permeable form of a metabolic substrate that can be oxidized in the tricarboxylic acid cycle to produce NADH to fuel electron transport and ATP production. Examples of such substrates include cell permeable form of pyruvate such as methylpyruvate. Cell death may be inhibited by providing a combination of the permeable form of the metabolic substrate and an autophagy inhibitor, such as those described above.

According to the disclosure, pharmaceutical compositions are provided for use in such methods of treating individuals who have a degenerative disease. The compositions may comprise a formulation of a metabolic substrate that can be oxidized in the tricarboxylic acid cycle to produce NADH to fuel electron transport and ATP production. The compositions may comprise a formulation of such a metabolic substrate in combination with an autophagy inhibitor. Pharmaceutical compositions may be formulated by one having ordinary skill in the art for delivery in the therapeutically effective dose for a chosen mode of administration as described in Example 2.

### Screening and discovery methods (Not part of the invention)

One aspect of the present disclosure provides methods of detecting or identifying an autophagy inhibitor. The methods may comprise contacting a growth factor-dependent, apoptosis-resistant cell that is deprived of growth factor with a test compound and measuring autophagy. The cell that is contacted with the test compound can be any growth factor-dependent, apoptosis-resistant cell including, but not limited to, a tumor cell, an immortalized cell, an undifferentiated cell, and the like.

As used herein, the term "apoptosis-resistant cell" refers to a cell that is unable to undergo apoptosis. In some embodiments, the apoptosis-resistant cell is deficient in functional Bak and Bax.

As used herein, the term "growth factor-dependent cell" refers to a cell that depends on at least one growth factor to grow. In some embodiments the growth factor is an interleukin. In some embodiments, the interleukin is IL-3. In some embodiments, the cell is grown in the presence of at least one growth factor. In some embodiments, the cell is grown or cultured in the presence of IL-3. In some embodiments, the cell is grown or cultured in the absence of IL-3. In some embodiments, the cell is grown in the presence of IL-3 and then IL-3 is withdrawn. In some embodiments, the withdrawal of IL-3 can induce autophagy.

As used herein, the term "tumor cell" refers to a cell that has been derived from a tumor. The tumor cell can be from a primary tumor or it can be from a tumor that has metastasized. The tumor cell can also be from a tumor cell line. Tumor cell lines are widely available and can be obtained from many companies including, but not limited to, ATCC (American Type Culture Collection, Rockville, MD).

As used herein, the term "immortalized cell" refers to a cell that does not under normal growth conditions undergo quiescence. An "immortalized cell" can in some embodiments, be a tumor cell. "Immortalized cells" can also be normal cells that have been transformed to become immortal. Examples of cells that can be immortalized include, but not limited to, embryonic fibroblasts, which include mouse embryonic fibroblasts (MEFs). Mouse embryonic fibroblasts undergo what is termed "crisis" that allows them to become immortalized.

As used herein, the term "undifferentiated cell" refers to a cell that can become differentiated or has the ability to become different types of cells depending on its environment and/or signals that the cell receives.

When growth factor is withheld from growth factor-dependent, apopotosis-resistant cells, the cells, which cannot undergo apoptosis, undergo autophagy. Initiation of autophagy is characterized by formation of autophagosomes which can be observed by detected localization of LC3. A preferred method of detecting localization of LC3 is by immunostaining with anti-LC3 antibodies.

The test compound can be contacted with a cell by any means that is available that puts the compound in contact with the cell. In some embodiments, the test compound is injected into the cell. If the cell is in an *in vitro* environment (e.g. cell culture) the test compound can be added to the media that the cell is growing in. The test compound can also be tested *in vivo* by administering the test compound to an animal. The test compound can be administered by any means available including, but not limited to, injection, orally, and the like.

In some embodiments, the methods of the present disclosure comprises contacting a test compound with the growth factor-dependent, apoptosis-resistant cell population that has been maintained in the absence of growth factor sufficient to induce autophagy, and measuring autophagy in the cells, as an indication of the effect of the test compound. In some embodiments, it is determined if the cells have undergone autophagy and is used as an indication of the effect of the test compound. In some embodiments, the effect of the test compound is compared to what occurs in the absence of any test compound.

In some embodiments of the present disclosure, the methods comprise contacting more than one test compounds, in parallel. In some embodiments, the methods comprises contacting 2, 3, 4, 5, 6, 7, 8, 9, 10, 15, 20, 25, 30, 40, 50, 100, 1000, at least 2, at least 5, at least 10, at least 50, at least 100, or at least 1000 test compounds in parallel. In some embodiments, the present invention uses High Throughput Screening of compounds and complete combinatorial libraries can be assayed, e.g., up to thousands of compounds. Methods of how to perform high throughput screenings are well known in the art. The methods can also be automated such that a robot can perform the experiments. The present invention can be adapted for the screening of large numbers of compounds, such as combinatorial libraries of compounds. Indeed, compositions and methods allowing efficient and simple screening of several compounds in short periods of time are provided. The instant methods can be partially or completely automated, thereby allowing efficient and simultaneous screening of large sets of compounds.

In some embodiments, the method of the present disclosure comprises the step of contacting a cell in the presence of a test compound. The cells can then be observed to determine if the test compound inhibits autophagy. A control may be provided in which the cell is not contacted with a test compound. If the cells contacted with the test compound inhibit autophagy then anti-autophagy activity is indicated for the test compound.

Positive and negative controls may be performed in which known amounts of test compound and no compound, respectively, are added to the assay. One skilled in the art would have the necessary knowledge to perform the appropriate controls.

The test compound can be any product in isolated form or in mixture with any other material (e.g., any other product(s)). The compound may be defined in terms of structure and/or composition, or it may be undefined. For instance, the compound may be an isolated and structurally-defined product, an isolated product of unknown structure, a mixture of several known and characterized products or an undefined composition comprising one or several products. Examples of such undefined compositions include for instance tissue samples, biological fluids, cell supernatants, vegetal preparations, etc. The test compound may be any organic or inorganic product, including a polypeptide (or a protein or peptide), a nucleic acid, a lipid, a polysaccharide, a chemical product, or any mixture or derivatives thereof. The compounds may be of natural origin or synthetic origin, including libraries of compounds.

In some embodiments, the activity of the test compound(s) is unknown, and the method of this invention is used to identify compounds exhibiting the selected property (e.g., autophagy inhibition). However, in some embodiments instances where the activity (or type of activity) of the test compound(s) is known or expected, the method can be used to further characterize the activity (in terms of specificity, efficacy, etc.) and/or to optimize the activity, by assaying derivatives of the test compounds.

The amount (or concentration) of test compound can be adjusted by the user, depending on the type of compound (its toxicity, cell penetration capacity, etc.), the number of cells, the length of incubation period, etc. In some embodiments, the compound can be contacted in the presence of an agent that facilitates penetration or contact with the cells. The test compound is provided, in some embodiments, in solution. Serial dilutions of test compounds may be used in a series of assays. In some embodiments, test compound(s) may be added at concentrations from 0.01 µM to 1 M. In some embodiments, a range of final concentrations of a test compound is from 10 µM to 100 µM.

In some embodiments, the method comprises measuring autophagy in the presence of the test compound. If the test compound is found to inhibit autophagy it is indicative that the test compound is an autophagy inhibitor. Autophagy can be measured by any means that demonstrates that the level of autophagy has been modulated (increased or decreased) in the presence of the test compound. Examples of how to measure autophagy include, but are not limited to determining LC3 localization or LC3 conversion.

LC3 localization can be viewed using any technique including, but not limited to, immunofluorescence. Under normal conditions (e.g. where autophagy is not occurring) LC3 dispersed throughout the cell when viewed using immunofluorescence. When autophagy occurs LC3 becomes localized at a distinct point(s) and can easily identified using immunofluorescence. LC3 can be visualized using an molecule that can bind to LC3. In some embodiments, an antibody is used to visualize LC3. In some embodiments, the antibody is a polyclonal or monoclonal antibody. One can measure immunofluorescence by any means including, for example, a microscope. Other techniques to measure immunofluorescence in a cell are known to one of skill in the art.

Autophagy can also be monitored by measuring LC3 conversion. During autophagy LC3 becomes localized to autophagosomes, which as discussed above can be measured and visualized using immunofluorescence. This migration to the autophagosomes can also be measured because LC3 undergoes a conversion from isoform I (LC3-I) to isoform II (LC3-II). This conversion can be monitored and/or measured by, for example, Western blot analysis. Accordingly, autophagy can be measured by measuring the conversion of LC3-I to LC3-II. A test compound would be said to be an autophagy inhibitor if the conversion of LC3-I to LC3-II is inhibited. In some embodiments, the conversion is inhibited by at least 10%, at least 20%, at least 30%, at least 40%, at least 50%, at least 60%, at least 70%, at least 80%, at least 90%, at least 95%, or at least 99%.

In some embodiments, the percent inhibition of autophagy is compared autophagy observed in the absence of the test compound.

As described above, the test compound can be contacted with a variety of cells to determine if it is an autophagy inhibitor. In some embodiments, the cell that is contacted with the test compound is unable to undergo apoptosis. In some embodiments the cell is deficient in the expression of the Bax gene, Bak gene, or both.

As used herein, the term "deficient in the expression of" refers to the gene or the product of a gene. The term "deficient in the expression of" can refer to status of the gene in the cell. In some embodiments, the cell is null for the gene in that it has no copies of the gene and is, therefore unable to express the gene. In some embodiments, the status of the gene or gene product is that it is mutated such that the gene is not expressed or that the gene product is not functional or has less function than the wild-type gene. Accordingly, a cell that is deficient in the expression of the Bax gene may have no Bax gene or the Bax gene may be mutated so that the Bax gene product is not functional or has less function than the wild-type gene.

In some embodiments, the cell that is contacted with the test compound is null for the Bax gene, Bak gene, or both. A non-limited example of a cell that is deficient for the expression of the Bax gene, Bak gene, or both is a mouse embryonic fibroblast that is deficient in bax and bak gene expression (Zong, et al., Genes & Development, 18:1272-1282 (2004)). This cell line is also described in U.S. Patent Application 20030091982, filed May 15, 2003. However, any cell can be used that is deficient for the Bax gene, Bak gene, or both.

In some embodiments, the cell that is contacted with the test compound is deficient in p53 gene expression. In some embodiments, a cell that is deficient in p53 gene expression can have the p53 gene deleted or be "null for p53" or the cell can comprise a mutant of p53 that inactivates the function of p53. In some embodiments, the p53 mutant is a dominant negative mutant or a temperature sensitive mutant. In some embodiments, the p53 mutation is a mutation that inhibits the binding of p53 to mdm2. In some embodiments, the p53 mutation inhibits the formation of a p53 tetramer.

Methods of creating a cell that is deficient in the expression of a particular gene or set of genes are known in the art. Examples include, but are not limited to those described in U.S. Patent Application 20030091982, siRNA, antisense oligonucleotides, and the like.

In some embodiments, the methods further comprise performing a control assay. In some embodiments, the control assay comprising contacting a cell with a negative or positive control and measuring, including, but not limited to, autophagy and the like. In some embodiments, the control compound is compared to the test compound. In some embodiments, the control compound is a negative control (e.g. a compound that does not inhibit autophagy). A negative control can also be the absence of a test compound or the vehicle (e.g. solvent) that the test compound is contacted with the cell. In some embodiments, the control compound is a positive control (e.g. a compound that inhibits autophagy. As discussed, herein, autophagy can be measured in the absence and the presence of the test compound.

The applicants provide the following examples.

### EXAMPLES

### Example 1

### Summary

In animals, cells are dependent on extracellular signals to prevent apoptosis. However, using growth factor-dependent cells from Bax/Bak-deficient mice, we demonstrate that apoptosis is not essential to limit cell autonomous survival. Following growth factor withdrawal, Bax^{-/-}Bak^{-/-} cells activate autophagy, undergo progressive atrophy, and ultimately succumb to death. These effects result from loss of the ability to take up sufficient nutrients to maintain cellular bioenergetics. Despite abundant extracellular nutrients, growth factor-deprived cells maintain ATP production from catabolism of intracellular substrates through autophagy. Autophagy is essential for maintaining cell survival following growth factor withdrawal and can sustain viability for several weeks. During this time, cells respond to growth factor readdition by rapid restoration of the ability to take up and metabolize glucose and by subsequent recovery of their original size and proliferative potential. Thus, growth factor signal transduction is required to direct the utilization of sufficient exogenous nutrients to maintain cell viability.

### Introduction

To determine if metabolic changes that result from growth factor withdrawal result from a primary effect of growth factor signal transduction or as a consequence of Bax and Bak activity, we examined the effects of growth factor withdrawal on IL-3-dependent Bax^{-/-}Bak^{-/-} cells. In addition to withdrawing from the cell cycle, cells cultured in the absence of growth factor underwent progressive atrophy. This atrophy correlated with the inability to utilize extracellular glucose and the induction of macroautophagy. Despite the abundance of oxidizable nutrients in the extracellular media, growth factor-deprived cells became dependent on the autophagic degradation of intracellular contents to maintain ATP production. Prevention of autophagy by RNAi-mediated suppression of autophagy genes or chemical inhibitors of autophagosome function led to rapid cell death. These data provide a demonstration that autophagy is critical for maintaining cell survival following growth factor withdrawal. While, by its very nature, macroautophagy is a self-limited survival strategy, it was able to promote growth factor-independent survival for several weeks. During this period, readdition of growth factor led to stimulation of glycolysis and complete cell recovery. Together, these data suggest that growth factor signal transduction is required to maintain a sufficient level of nutrient utilization to support the survival of mammalian cells.

### Experimental Procedures

### Cell Culture, Reagents and Inhibition Assays

Immortalized IL-3 dependent cells were obtained from the bone marrow of two independent Bax^{-/-}Bak^{-/-} mice using previously established protocols. Subsequent experiments using these cells were performed in complete media consisting of: RPMI 1640 medium (Invitrogen) supplemented with 10% heat inactivated fetal bovine serum (Gemini), 10 units/mL penicillin/streptomycin and 2 mM L-glutamine (Invitrogen), 50 µm β-mercaptoethanol (Sigma) and 10 mM HEPES (Invitrogen). For cells grown in the presence of IL-3, the complete medium was supplemented with 3.5 ng/mL of murine recombinant IL-3 (BD Pharmingen). For IL-3 withdrawal experiments, cells were washed three times in medium without IL-3 and serum. After the final wash, cells were resuspended in complete medium without IL-3. Medium for cells cultured in the absence of IL-3 was replaced with fresh complete medium without IL-3 every 10 days. To inhibit autophagy, cells cultured in the absence of IL-3 were pelleted and resuspended in IL-3 deficient medium containing either 5 mM 3-methyladenine (Sigma) or 10 µM chloroquine (Sigma) in the presence or absence of 10 mM methylpyruvate (Sigma). Cell size and number was assessed by using a Coulter Z2 particle analyzer. For readdition experiments, cells grown in the absence of IL-3 at the indicated time points were pelleted by centrifugation and resuspended in complete media containing 3.5 ng/mL IL-3. The FL5.12 cell line was maintained in complete media supplemented with 0.35 ng/mL IL-3.

### Primary Bone Marrow Cultures

Primary cell cultures were prepared from Bax^{-/-}Bak^{-/-} bone marrow cells and were cultured in the presence or absence of 3.5 ng/mL recombinant IL-3 in RPMI 1640 medium (Invitrogen) supplemented with 10% heat inactivated fetal bovine serum (Gemini), 10 units/mL penicillin/streptomycin and 2 mM L-glutamine (Invitrogen), 50 µm β-mercaptoethanol (Sigma) and 10 mM HEPES (Invitrogen). Non-adherent cells were collected every two days and resuspended in fresh media with or without IL-3.

### Constructs, Retroviral Infections and RNAi

Bax and Bak were subcloned into pBabe-IRES GFP containing retroviral vectors and transfected into the Phoenix packaging cell line. Viral supernants were used to infect Bax^{-/-}Bak^{-/-} cells in the presence of 10 ng/mL polybrene and 3.5 ng/mL IL-3. Ten days post-infection, single GFP-positive cells were FACS sorted into 96 well plates and expanded as required. Short hairpin RNA constructs were generated against *ATG5* using previously established protocols. Briefly, hairpin specific primers were used in a PCR reaction using pEF6-hU6 as a template. The PCR products were subcloned into TOPO-TA, digested with BamHI and EcoRV and ligated into pKD-GFP. The *ATG*5 sense primers were: hp2 5' GGC ATT ATC CAA TTG GTT TA, hp7 5' GCA GAA CCA TAC TAT TTG CT. Eight micrograms of DNA were introduced into Bax^{-/-}Bak^{-/-} cells by Nucleofector transfection (Amaxa) using program T20. Twenty four to 48 hours post-transfection, GFP-positive cells were FACS sorted and the resulting population was placed in complete medium supplemented with or without IL-3. Both an oligoribonucleotide for *ATG7* (Yu et al., 2004) and a control oligoribonucleotide were synthesized with an N-terminally-conjugated fluorescein-5-isothiocyanate (FITC) tag (Invitrogen). Each ribonucleotide (0.5 nmol) was introduced into Bax^{-/-}Bak^{-/-} cells by Nucleofector transfection (Amaxa) using program T20. Twenty four to 48 hours post-transfection FITC-positive cells were FACS sorted and the resulting population was placed in complete medium supplemented with or without IL-3.

### Membrane Potential and Cell Death Assays

For measurement of mitochondrial membrane potential, cells were incubated for 30 minutes with 50 nM of tetramethyl rhodamine ethyl ester (TMRE; Molecular Probes) in the presence or absence of 50 µM CCCP (Sigma). Viability was performed by incubating cells with annexin V conjugated fluorescein isothiocyanate (BD Pharmingen) in buffer containing 1 µg/mL propidium iodide (Molecular Probes) followed by FACS analysis. DNA fragmentation assay was performed as previously described.

### Electron Microscopy

Cells were fixed with 2.5% glutaraldehyde/2% formaldehyde with 0.1 M sodium cacodylate and stored at 4°C until embedding. Cells were post-fixed with 2% osmium tetroxide followed by an increasing gradient dehydration step using ethanol and propylene oxide. Cells were then embedded in LX-112 medium (Ladd) and sections were cut ultrathin (90 nm), placed on uncoated copper grids and stained with 0.2% lead citrate and 1% uranyl acetate. Images were examined with a JEOL-1010 electron microscope (JEOL) at 80 kV. For quantitation of autophagosomes, the data obtained from a minimum of 50 independent cells was averaged (mean ± S.D.)

### Immunoblotting, Immunofluorescence and Surface Staining

Cells were lysed in RIPA buffer and proteins were subjected to SDS-PAGE on 4-12%NuPAGE gels (Invitrogen). Antibodies (all 1:1000 dilution) used were: GLUT1 (Research Diagnostic Inc.), calreticulin (StressGen), STAT3 (Cell Signalling), actin (Sigma), LC3 (gift from T. Yoshimori), ATG5 (gift from N. Mizushima), Bax (Santa Cruz) and Bak (Upstate Technologies). For immunofluorescence staining, cells were fixed in 4% paraformaldehyde and permeabilized with 0.1% Triton X-100, washed 3 times (PBS containing 0.01% Triton X-100 and 10% FBS), followed by incubation with anti-rabbit LC3. A 1:100 dilution of Alexa 488 (Molecular Probes) secondary antibody was used. Images were captured using a Zeiss 510 confocal microscope. For surface analysis, cells were fixed in 4% paraformaldehyde, stained with 1:100 biotin-anti-IL-3 alpha chain receptor (BD Pharmingen) and a 1:100 dilution of streptavidin conjugated fluorescein isothiocyanate (BD Pharmingen) followed by FACS analysis.

### Measurement of Glycolysis and ATP

The conversion of 5-³H-glucose to ³H₂O was used to measure the glycolytic rate. The level of ATP was measured as described previously.

### Results

### Growth Factor Withdrawal Results in Progressive Atrophy of Bax^{-/-}Bak^{-/-} Cells

To study the effects of growth factor withdrawal on cells lacking the intrinsic apoptotic pathway, immortalized interleukin-3 (IL-3) dependent cell lines were generated from the bone marrow of Bax^{-/-}Bak^{-/-} mice. These cells failed to undergo apoptosis following growth factor withdrawal (Figure 1), but remained dependent on IL-3 for proliferation in culture. Transfection of either Bax or Bak fully restored apoptosis in these cells in response to IL-3 withdrawal with comparable kinetics to that observed in wild-type IL-3-dependent cells (Figures 1A and 1B). Following IL-3 withdrawal, the Bax^{-/-}Bak^{-/-} cells exited from the cell cycle and the cell number in the culture did not change during the first several weeks (Figure 1D). Although the initial decline in cell size that occurs in the first two days after growth factor withdrawal results from the arrest of the cells in the G₀/G₁ phase of the cell cycle, cell size continued to decline at subsequent time points and no stable cell size was achieved as measured by either cell size or protein content (Figure 1E and data not shown). Beginning at approximately 12 weeks, cell number and viability also began to decline and >95% of cells were dead by 24 weeks of culture (Figures 1C and 1D).

### Autophagosome Formation is Induced by Growth Factor Withdrawal

Cells grown in the presence of IL-3 were highly glycolytic (Figure 2A). In contrast, glycolysis declined rapidly following IL-3 withdrawal and there was a time dependent loss of GLUT1, the major glucose transporter expressed on these cells (Figure 2B). Coincident with the decline in glycolysis there was a decline in mitochondrial membrane potential (Figure 2C). Cellular ATP levels also fell, but the decline in glucose transporter expression was greater than that expected based on the ATP decline (Figure 2D). This suggested that cells were utilizing alternative substrates to maintain their bioenergetics. Furthermore, the continued decline in cell size of the G₀/G₁ arrested cells following growth factor withdrawal suggested the possibility that cells were utilizing macroautophagy to catabolize intracellular substrates to maintain their survival. These observations prompted a characterization of the cells during growth factor withdrawal by electron microscopy. By 48 hours after growth factor withdrawal, early autophagosomes were visible in the cytosol of the cells (Figure 3Aa-c). The presence of autophagosomes when quantitated by electron micrographs was significantly increased in the IL-3 deprived cells in comparison to cells maintained in IL-3 (Figure 3Ad, p<0.001). To confirm the extent and specificity of this autophagosome induction, the cells were stained with an antibody specific for the mammalian homologue of the yeast Atg8 protein, microtubule-associated protein-1 light chain-3 (LC3). LC3 becomes physically associated with forming autophagic vesicles and is a well-characterized marker for autophagosome formation. Using confocal microscopy, we observed a redistribution of LC3 from diffuse cytoplasmic staining in cells grown in the presence of IL-3 (Figure3Ba) to discrete vesicular structures following IL-3 withdrawal (Figure 3Bb). This redistribution was confirmed biochemically by Western blot analysis. The intracellular LC3 underwent a conversion from the LC3-I isoform to the LC3-II isoform that is specific for autophagosomes (Figure 3C, lane 1 versus lane 4).

### Inhibition of Autophagy Leads to Cell Death

The ability of cells to initiate autophagosome formation is dependent on the ATG12-ATGS complex. To test whether macroautophagy plays a role in maintaining growth factor independent cell survival, shRNA against *ATG5* were introduced into the IL-3 dependent cells. Cells transfected with two independent shRNA constructs against *ATG5* (hp-2 and hp-7) or a control had no effect on the size or viability of cells grown in the presence of IL-3 (data not shown). In contrast, if cells transfected with *ATG5* hairpins were withdrawn from IL-3, their viability began to decline at 48 hours after withdrawal and virtually all cells were dead by 96 hours (Figure 3D). The onset and rapidity of decline in cell viability correlated with the extent of ATG5 protein suppression by shRNA (Figure 3D), with the absence of autophagic processing of LC3 in growth factor-deprived cells in which *ATG5* is suppressed (Figure 3C, lane 4 versus lane 5 & 6), and a statistically significant reduction in autophagosomes observed in electron micrographs at 48 hours after IL-3 withdrawal (data not shown). Similar results on cell survival were obtained when autophagy was suppressed with siRNA against *ATG7* (Figure 3E).

While macroautophagy in yeast and plant cells is required to promote cell survival in the absence of nutrients, the macroautophagy observed following IL-3 deprivation occurred in the presence of abundant extracellular nutrients. The IL-3-deprived cells were maintained in complete RPMI medium supplemented with 10% serum and the medium was replaced every 10 days. The medium removed from these cultures was not nutrient deficient since it supported proliferative expansion of the parental Bax^{-/-}Bak^{-/-} cells when supplemented with IL-3 (data not shown). Therefore, macroautophagy in Bax^{-/-}Bak^{-/-} cells was induced by growth factor withdrawal and not by a lack of nutrients in the extracellular environment.

### Macroautophagy is an Ongoing Process in Surviving Growth Factor-Deprived Cells

To determine if macroautophagy persisted during the weeks the cells survived growth factor deprivation, electron micrographs were obtained from later time points following IL-3 withdrawal (Figure 4). In the weeks following IL-3 deprivation, the cytoplasm of the cells became progressively replaced by vesicular structures, some of which contained residual remnants of degraded organelles and others the characteristics of lysosomes (Figure 4Aa-d). High power transmission electron microscopy images demonstrate continued presence of autophagosomes and late autophagosomes fusing with lysosomes (Figure 4Ae and 4Af). These structures were quantitatively increased in IL-3 deprived cells even after 6 weeks in culture (Figure 4B, p<0.001). The continued presence of autophagosomes was confirmed by the persistent vesicular staining pattern of LC3 (Figure 4Ca versus 4Cb). This persistent formation of autophagosomes correlated with a progressive reduction of definitive intracellular organelles. By six weeks, ribosomes were difficult to find and the Golgi/ER network was not observed in any of the sections examined. The few mitochondria that remained were perinuclear in distribution and highly condensed. The nucleus displayed a reduced number of nuclear pores, reduced nucleolar size and a more organized heterochromatin.

### The TCA-Substrate Methylpyruvate Maintains the Survival of Growth Factor-Deprived Cells Treated with Inhibitors of Autophagosome/Lysosome Function

We next tested whether the continued degradation of metabolic substrates within the autophagosome/lysosome system was required to maintain cell viability at these late time points after growth factor withdrawal. Existing shRNA transfection methods proved ineffective in cells that had undergone prolonged growth factor withdrawal; therefore we used two independent and widely used inhibitors of macroautophagy, 3-methyladenine (3-MA) and chloroquine (CQ) to block autophagy. Treatment with either 5 mM 3-MA or 10 µM CQ had no significant effect on survival of cells grown in the presence of IL-3 (Figure 5). However, when cells deprived of growth factor for 6 weeks were cultured with 3-MA or CQ cell viability began to decline within 18 hours. By 48 hours, treatment with 3-MA or CQ had resulted in the death of 72% and 82% of the cells in the cultures respectively. These effects of 3-MA or CQ were dose dependent (data not shown). Cell death induced by 3-MA and CQ correlated with a reduction in the number of autophagosomes as evidenced by the redistribution of LC3 from highly localized punctate staining to diffuse cytoplasmic staining (Figure 5B, b versus d and b versus f). This death did not appear to be apoptotic in nature. DNA extracted from the dying cells lacked oligonucleosomal length fragments characteristic of apoptosis (Figure 5C). In addition, the dying cells lost their capacity to exclude propidium iodide prior to becoming annexin V positive (Figure 5D) and caspase inhibitors failed to prevent this death (data not shown).

Since autophagy is required in yeast to provide mitochondria with substrates to maintain oxidative phosphorylation during nutrient deprivation, we tested whether the cell death observed following 3-MA and CQ treatment could be reversed by supplying the cell with an alternative metabolic substrate. A cell-permeable form of pyruvate, methylpyruvate (MP), was added to the cultures at the time of 3-MA or CQ treatment. Once internalized, this substrate can be oxidized in the tricarboxylic acid cycle to produce NADH to fuel electron transport and ATP production. The addition of methylpyruvate suppressed the death observed in response to 3-MA and CQ in a time and dose-dependent fashion (Figure 5E and data not shown).

To confirm that suppression of autophagy led to compromised cellular bioenergetics that are restored by methylpyruvate, ATP levels were measured in growth factor-deprived cells treated with 3-MA or CQ. After 8 hours of addition of either drug, there was no observable cell death in the culture. Despite this, the IL-3 deprived cells treated with 3-MA (Figure 5F) or CQ (data not shown) experienced a dramatic decline in ATP levels. The ATP decline could be suppressed by supplying a cell-permeant bioenergetic substrate, methylpyruvate. Despite an abundance of oxidizable substrates in the medium including serum lipids, amino acids, and glucose, the IL-3-withdrawn cells were unable to utilize them to maintain ATP production.

### Growth Factor Regulates Autophagy in Primary Bax^{-/-}Bak^{-/-} Bone Marrow Cells

The properties described above were reproduced in two independently-derived IL-3-dependent Bax^{-/-}Bak^{-/-} cell lines. To determine whether these results also applied to primary cells, we isolated Bax^{-/-}Bak^{-/-} bone marrow cells from mice and cultured the cells in the presence or absence of IL-3 for 14 days immediately following isolation. Consistent with the data from the immortalized IL-3 dependent cells, bone marrow cells grown in the absence of IL-3 were smaller, displayed LC3-positive autophagosomes in their cytoplasm, and were dependent on autophagy to support cell survival and ATP production (Figure 6A and 6B). Many cells in the culture retained the ability to grow and proliferate when IL-3 was restored.

### Growth Factor Readdition Restores Glycolysis and Cell Growth/Proliferation

In unicellular organisms, an important feature of autophagic maintenance of cell survival is the ability of the cells to recover and proliferate if nutrients reappear. Despite the loss of cell surface nutrient transporters, the absence of an observable Golgi/ER, and a profound decline in total protein content, the cells cultured in the absence of IL-3 had higher levels of surface IL-3 receptor (Figure 7A) than cells grown in the presence of IL-3. In addition, the IL-3 receptor-activated transcription factor STAT3, a known regulator of GLUT1 expression, was still expressed (data not shown). Therefore, we determined whether the ability of IL-3 to regulate glucose uptake and metabolism was intact. Within 4 hours of IL-3 readdition the glycolytic rate of the cells increased almost 5 fold and by 24 hours increased to levels comparable to those of cells grown in the presence of IL-3 (Figure 7B).

Although glycolysis recovered rapidly following IL-3 readdition, cells did not regain their ability to grow and proliferate immediately. The recovery time for cell size and proliferation varied depending on the length of time the cells had been deprived of IL-3. Virtually all cells deprived of IL-3 for 2 or 6 weeks were ultimately able to recover as measured by cell growth and proliferation when placed in IL-3 containing medium (Figures 7C and 7D). However, the kinetics of recovery were dramatically different depending on the length of time the cells were deprived of IL-3. All of the cells in both cultures began to grow in size in response to IL-3 (Figure 7E). After only 3 days of IL-3, the average cell in the 2 week-deprived cultures had grown from 276 fL to 439 fL. In contrast, even after 11 days of IL-3, the average cell in the 6 week-deprived cultures had only grown from 241 fL to 353 fL (Figure 7E). In comparison to the cells in the 2 week-deprived cultures, it took over a week longer for cells in the 6 week-deprived cultures to begin to divide and accumulate. When fully recovered, both populations had a size distribution and doubling time indistinguishable from the starting population.

### Discussion

### Growth Factors Regulate Exogenous Nutrient Utilization And Survival

The above results suggest that in addition to regulating apoptosis, growth factors promote cell survival by maintaining the ability of cells to take up sufficient nutrients to maintain ATP production and to support self-sustaining macromolecular biosynthesis (anapleurosis). Previous work has suggested that extracellular ligands primarily regulate anabolic processes, hence the collective term growth factors. Anapleurotic processes that are self-maintaining have been thought to be intrinsically controlled by cells because the extracellular environment of a healthy animal has an abundant supply of extracellular nutrients. However, the present work suggests that hematopoietic cells depend on extracellular signals like IL-3 for self-maintenance even when cultured in otherwise complete medium. In the absence of signal transduction by the lineage-specific factor IL-3, receptor-expressing cells undergo progressive atrophy and must use macroautophagy to support a sufficient level of ATP production to maintain viability. Such cellular catabolism can promote cell survival for a number of weeks in the absence of extracellular signals, but this mechanism of promoting cell autonomous survival is necessarily self-limited and ultimately results in death unless growth factor is resupplied. Thus, growth factor signal transduction is absolutely required to maintain hematopoietic cell survival.

### Macroautophagy is a Conserved But Self-Limited Survival Mechanism

Based on the results, macroautophagy appears to be an evolutionarily conserved survival strategy. Macroautophagy can support growth factor-independent cell survival of hematopoietic cells for several weeks. Readdition of growth factor during this period leads to cell recovery. Similarly, in both plants and yeast, survival in response to nutrient deprivation is dependent on macroautophagy. Macroautophagy can support survival for several weeks during which time nutrient readdition supports recovery. Thus, it appears eukaryotic cells share a common survival pathway that promotes cell-autonomous survival in the face of starvation and/or neglect. Animal cells may have evolved an apoptotic response in part to limit this form of cell-autonomous survival. Nevertheless, as previously demonstrated in unicellular organisms, macroautophagy is a self-limited survival strategy and ultimately will result in cell death if not reversed.

The catabolic effects of macroautophagy do have significant consequences for the cell. Although cells retain the ability to rapidly respond to growth factor stimulation by upregulating glycolysis, their ability to proliferate in response to growth factor stimulation becomes impaired. For example, cells deprived of IL-3 for 6 weeks take 14 days following IL-3 readdition to reenter S phase. During this time, they must reverse the catabolic effects of macroautophagy by resynthesizing cellular organelles and cell cycle regulatory proteins.

In contrast to the role of autophagy in promoting cell survival either during nutrient or growth factor deprivation as described here, cell death associated with autophagy has been observed in response to viral infection, ER stress, toxins and chemotherapy drugs. In some of these cases, inhibition of autophagy prevents cells from undergoing non-apoptotic death. These results do not argue against a protective role for autophagy during cellular stress since autophagy may be a strategy to limit cell death by clearance of damaged organelles which can activate apoptosis. However, this compensatory mechanism if overactivated may compromise the ability of a cell to ultimately recover if autophagy-mediated clearance results in complete elimination of an essential organelle.

The concept of nutrient starvation-induced autophagy and its essential role in survival originated from studies in yeast and has begun to be extended to multicellular organisms. In response to starvation, *C. elegans* larvae enter dauer, a latent developmental state. Inactivation of *ATG* homologs disrupts normal dauer formation. Recent results suggest that fruit flies also require autophagy to adapt to organismal nutrient starvation. In plants with mutant autophagy genes, nitrogen starvation induces development defects including accelerated senescence and enhanced chlorosis. These effects on survival are observed in *D. discoideum* where defective fruiting bodies are formed in autophagy mutants in response to nutrient starvation. Autophagy is also critical to maintain the survival of neonatal mice during the period between birth and the establishment of their ability to be nursed effectively by their mothers (Kuma et al., 2004). However, while mammalian cells normally have ample nutrient resources in their extracellular environment in the fed state, the present data demonstrate that growth factor withdrawal results in the loss of the cellular ability to utilize extracellular nutrients to maintain themselves. When conserved components of the autophagic program are inactivated, cells succumb to cell death despite abundant extracellular nutrients.

### Implications for the Regulation of Cell Death During Development

Although apoptosis is not absolutely required for growth factor regulation of cell survival, these data may also help explain why apoptosis is so important in the development of animals. Because macroautophagy can maintain cell survival for a number of weeks following growth factor withdrawal, the time scale of growth factor withdrawal-induced death in the absence of apoptosis is too slow to permit effective culling of extraneous cells produced during embryonic patterning. This is most clearly seen in mice with genetic defects in core apoptotic genes such as Bax^{-/-}Bak^{-/-}, APAF-1^{-/-}, caspase 3^{-/-}, or caspase 9^{-/-} mice. These deficiencies are associated with perinatal death resulting from the failure to eliminate the excess neurons produced during the development of the central nervous system (CNS).

Together, these data suggest that apoptosis is not the only mechanism by which animals can limit the survival of unwanted cells. Elimination of extracellular factors on which cells depend to maintain anapleurotic reactions and bioenergetics will be as effective at eliminating them, albeit more slowly. These results may explain how other multicellular organisms such as plants can limit the survival of cells that accumulate in excess despite the apparent lack of homologues of the central apoptotic control genes in their genomes.

### Example 2

Suitable pharmaceutical carriers are described in the most recent edition of Remington's Pharmaceutical Sciences, A. Osol, a standard reference text in this field.

Administering the pharmaceutical composition can be effected or performed using any of the various methods known to those skilled in the art. Systemic formulations include those designed for administration by injection, e.g. subcutaneous, intravenous, intramuscular, intrathecal or intraperitoneal injection, as well as those designed for transdermal, transmucosal, oral or pulmonary administration.

For injection, the compounds of the invention may be formulated in aqueous solutions, preferably in physiologically compatible buffers such as Hanks's solution, Ringer's solution, or physiological saline buffer. The solution may contain formulatory agents such as suspending, stabilizing and/or dispersing agents. Injectables are sterile and pyrogen free. Alternatively, the compounds may be in powder form for constitution with a suitable vehicle, e.g., sterile pyrogen-free water, before use. For transmucosal administration, penetrants appropriate to the barrier to be permeated are used in the formulation. Such penetrants are generally known in the art.

For parenteral administration, the autophagy inhibitor and/or anti-cancer drug and or combination formulation thereof can be, for example, formulated as a solution, suspension, emulsion or lyophilized powder in association with a pharmaceutically acceptable parenteral vehicle. Examples of such vehicles are water, saline, Ringer's solution, dextrose solution, 5% human serum albumin, Ringer's dextrose, dextrose and sodium chloride, lactated Ringer's and fixed oils, polyethylene glycol, polyvinyl pyrrolidone, lecithin, arachis oil or sesame oil.. Liposomes and nonaqueous vehicles such as fixed oils may also be used. The vehicle or lyophilized powder may contain additives that maintain isotonicity (e.g., sodium chloride, mannitol) and chemical stability (e.g., buffers and preservatives). The formulation is sterilized by commonly used techniques. Parenteral dosage forms may be prepared using water or another sterile carrier. For example, a parenteral composition suitable for administration by injection is prepared by dissolving 1.5% by weight of active ingredient in 0.9% sodium chloride solution. Alternatively, the solution can be lyophilised and then reconstituted with a suitable solvent just prior to administration.

Pharmaceutically acceptable carriers are well known to those skilled in the art and include, but are not limited to, 0.01-0.1 M and preferably 0.05 M phosphate buffer or 0.8% saline. Intravenous carriers include fluid and nutrient replenishers, electrolyte replenishers such as those based on Ringer's dextrose, and the like. Additionally, such pharmaceutically acceptable carriers can be aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, ethanol, alcoholic/aqueous solutions, glycerol, emulsions or suspensions, including saline and buffered media.

The pharmaceutical compositions can be prepared using conventional pharmaceutical excipients and compounding techniques. Oral dosage forms may be elixers, syrups, tablets , pills, dragees, capsules, liquids, gels, syrups, slurries, suspensions and the like, for oral ingestion by a patient to be treated. The typical solid carrier may be an inert substance such as lactose, starch, glucose, cellulose preparations such as maize starch, wheat starch, rice starch, potato starch, gelatin, gum tragacanth, methyl cellulose, hydroxypropylmethyl-cellulose, sodium carboxymethylcellulose, and/or polyvinylpyrrolidone (PVP); granulating agents; binding agents, magnesium sterate, dicalcium phosphate, mannitol and the like. A composition in the form of a capsule can be prepared using routine encapsulation procedures. For example, pellets containing the active ingredient can be prepared using standard carrier and then filled into a hard gelatin capsule; alternatively, a dispersion or suspension can be prepared using any suitable pharmaceutical carrier(s), for example, aqueous gums, celluloses, silicates or oils and the dispersion or suspension then filled into a soft gelatin capsule. Typical liquid oral excipients include ethanol, glycerol, glycerine, non-aqueous solvent, for example, polyethylene glycol, oils, or water with a suspending agent, preservative, flavoring or coloring agent and the like. All excipients may be mixed as needed with disintegrants, diluents, lubricants, and the like using conventional techniques known to those skilled in the art of preparing dosage forms. If desired, disintegrating agents may be added, such as the cross-linked polyvinylpyrrolidone, agar, or alginic acid or a salt thereof such as sodium alginate. If desired, solid dosage forms may be sugar-coated or enteric-coated using standard techniques. For oral liquid preparations such as, for example, suspensions, elixirs and solutions, suitable carriers, excipients or diluents include water, glycols, oils, alcohols, etc. Additionally, flavoring agents, preservatives, coloring agents and the like may be added.

For buccal administration, the compounds may take the form of tablets, lozenges, and the like formulated in conventional manner. The compounds may also be formulated in rectal or vaginal compositions such as suppositories or enemas. A typical suppository formulation comprises a binding and/or lubricating agent such as polymeric glycols, glycerides, gelatins or cocoa butter or other low melting vegetable or synthetic waxes or fats. For administration by inhalation, the compounds for use according to the present invention are conveniently delivered in the form of an aerosol spray from pressurized packs or a nebulizer, with the use of a suitable propellant, e.g., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of e.g. gelatin for use in an inhaler or insufflator may be formulated containing a powder mix of the compound and a suitable powder base such as lactose or starch.

The formulations may also be a depot preparation which can be administered by implantation (for example subcutaneously or intramuscularly) or by intramuscular injection. In such embodiments, the compounds may be formulated with suitable polymeric or hydrophobic materials (for example as an emulsion in an acceptable oil) or ion exchange resins, or as sparingly soluble derivatives, for example, as a sparingly soluble salt.

Alternatively, other pharmaceutical delivery systems may be employed. Liposomes and emulsions are well known examples of delivery vehicles that may be used. Certain organic solvents such as dimethylsulfoxide also may be employed, although usually at the cost of greater toxicity. Additionally, the compounds may be delivered using a sustained-release system, such as semipermeable matrices of solid polymers containing the therapeutic agent. Various of sustained-release materials have been established and are well known by those skilled in the art. Sustained-release capsules may, depending on their chemical nature, release the compounds for a few weeks up to over 100 days. Depending on the chemical nature and the biological stability of the therapeutic reagent, additional strategies for protein stabilization may be employed.

The compounds used in the invention may also be formulated for parenteral administration by bolus injection or continuous infusion and may be presented in unit dose form, for instance as ampoules, vials, small volume infusions or pre-filled syringes, or in multi-dose containers with an added preservative.

Preservatives and other additives can also be present, such as, for example, antimicrobials, antioxidants, chelating agents, inert gases and the like. All carriers can be mixed as needed with disintegrants, diluents, granulating agents, lubricants, binders and the like using conventional techniques known in the art.

The pharmaceutical compositions described above may be administered by any means that enables the active agent to reach the agent's site of action in the body of the individual. The dosage administered varies depending upon factors such as: pharmacodynamic characteristics; its mode and route of administration; age, health, and weight of the recipient; nature and extent of symptoms; kind of concurrent treatment; and frequency of treatment.

The amount of compounds administered will be dependent on the activity of the compounds subject being treated, on the subject's weight, the severity of the affliction, the manner of administration and the judgment of the prescribing physician. In some embodiments, the dosage range would be from about 1 to 3000 mg, in particular about 10 to 1000 mg or about 25 to 500 mg, of active ingredient, in some embodiments 1 to 4 times per day, for an average (70 kg) human. Generally, activity of individual compounds used in the invention will vary.

Dosage amount and interval may be adjusted individually to provide plasma levels of the compounds which are sufficient to maintain therapeutic effect. Usually, a dosage of the active ingredient can be about 1 microgram to 100 milligrams per kilogram of body weight. In some embodiments a dosage is 0.05 mg to about 200 mg per kilogram of body weight.. In another embodiment, the effective dose is a dose sufficient to deliver from about 0.5 mg to about 50 mg. Ordinarily 0.01 to 50 milligrams, and in some embodiments 0.1 to 20 milligrams per kilogram per day given in divided doses 1 to 6 times a day or in sustained release form is effective to obtain desired results. In some embodiments, patient dosages for administration by injection range from about 0.1 to 5 mg/kg/day, preferably from about 0.5 to 1 mg/kg/day. Therapeutically effective serum levels may be achieved by administering multiple doses each day. Treatment for extended periods of time will be recognized to be necessary for effective treatment.

In some embodiments, the route may be by oral administration or by intravenous infusion. Oral doses generally range from about 0.05 to 100 mg/kg, daily. Some compounds used in the invention may be orally dosed in the range of about 0.05 to about 50 mg/kg daily, while others may be dosed at 0.05 to about 20 mg/kg daily. Infusion doses can range from about 1.0 to 1.0 x 10⁴ microgram/kg/min of inhibitor admixed with a pharmaceutical carrier over a period ranging from several minutes to several days.

### Example 3

The National Cancer Institute alphabetical list of cancer includes: Acute Lymphoblastic Leukemia, Adult; Acute Lymphoblastic Leukemia, Childhood; Acute Myeloid Leukemia, Adult; Adrenocortical Carcinoma; Adrenocortical Carcinoma, Childhood; AIDS-Related Lymphoma; AIDS-Related Malignancies; Anal Cancer; Astrocytoma, Childhood Cerebellar; Astrocytoma, Childhood Cerebral; Bile Duct Cancer, Extrahepatic; Bladder Cancer; Bladder Cancer, Childhood; Bone Cancer, Osteosarcoma/Malignant Fibrous Histiocytoma; Brain Stem Glioma, Childhood; Brain Tumor, Adult; Brain Tumor, Brain Stem Glioma, Childhood; Brain Tumor, Cerebellar Astrocytoma, Childhood; Brain Tumor, Cerebral Astrocytoma/Malignant Glioma, Childhood; Brain Tumor, Ependymoma, Childhood; Brain Tumor, Medulloblastoma, Childhood; Brain Tumor, Supratentorial Primitive Neuroectodermal Tumors, Childhood; Brain Tumor, Visual Pathway and Hypothalamic Glioma, Childhood; Brain Tumor, Childhood (Other); Breast Cancer; Breast Cancer and Pregnancy; Breast Cancer, Childhood; Breast Cancer, Male; Bronchial Adenomas/Carcinoids, Childhood; Carcinoid Tumor, Childhood; Carcinoid Tumor,Gastrointestinal; Carcinoma, Adrenocortical; Carcinoma, Islet Cell; Carcinoma of Unknown Primary; Central Nervous System Lymphoma, Primary; Cerebellar Astrocytoma, Childhood; Cerebral Astrocytoma/Malignant Glioma, Childhood; Cervical Cancer; Childhood Cancers; Chronic Lymphocytic Leukemia; Chronic Myelogenous Leukemia; Chronic Myeloproliferative Disorders; Clear Cell Sarcoma of Tendon Sheaths; Colon Cancer; Colorectal Cancer, Childhood; Cutaneous T-Cell Lymphoma; Endometrial Cancer; Ependymoma, Childhood; Epithelial Cancer, Ovarian; Esophageal Cancer; Esophageal Cancer, Childhood; Ewing's Family of Tumors; Extracranial Germ Cell Tumor, Childhood; Extragonadal Germ Cell Tumor; Extrahepatic Bile Duct Cancer; Eye Cancer, Intraocular Melanoma; Eye Cancer, Retinoblastoma; Gallbladder Cancer; Gastric (Stomach) Cancer; Gastric (Stomach) Cancer, Childhood; Gastrointestinal Carcinoid Tumor; Germ Cell Tumor, Extracranial, Childhood; Germ Cell Tumor, Extragonadal; Germ Cell Tumor, Ovarian; Gestational Trophoblastic Tumor; Glioma, Childhood Brain Stem; Glioma, Childhood Visual Pathway and Hypothalamic; Hairy Cell Leukemia; Head and Neck Cancer; Hepatocellular (Liver) Cancer, Adult (Primary); Hepatocellular (Liver) Cancer, Childhood (Primary); Hodgkin's Lymphoma, Adult; Hodgkin's Lymphoma, Childhood; Hodgkin's Lymphoma During Pregnancy; Hypopharyngeal Cancer; Hypothalamic and Visual Pathway Glioma, Childhood; Intraocular Melanoma; Islet Cell Carcinoma (Endocrine Pancreas); Kaposi's Sarcoma; Kidney Cancer; Laryngeal Cancer; Laryngeal Cancer, Childhood; Leukemia, Acute Lymphoblastic, Adult; Leukemia, Acute Lymphoblastic, Childhood; Leukemia, Acute Myeloid, Adult; Leukemia, Acute Myeloid, Childhood; Leukemia, Chronic Lymphocytic; Leukemia, Chronic Myelogenous; Leukemia, Hairy Cell; Lip and Oral Cavity Cancer; Liver Cancer, Adult (Primary); Liver Cancer, Childhood (Primary); Lung Cancer, Non-Small Cell; Lung Cancer, Small Cell; Lymphoblastic Leukemia, Adult Acute; Lymphoblastic Leukemia, Childhood Acute; Lymphocytic Leukemia, Chronic; Lymphoma, AIDS-Related; Lymphoma, Central Nervous System (Primary); Lymphoma, Cutaneous T-Cell; Lymphoma, Hodgkin's, Adult; Lymphoma, Hodgkin's, Childhood; Lymphoma, Hodgkin's During Pregnancy; Lymphoma, Non-Hodgkin's, Adult; Lymphoma, Non-Hodgkin's, Childhood; Lymphoma, Non-Hodgkin's During Pregnancy; Lymphoma, Primary Central Nervous System; Macroglobulinemia, Waldenström's; Male Breast Cancer; Malignant Mesothelioma, Adult; Malignant Mesothelioma, Childhood; Malignant Thymoma; Medulloblastoma, Childhood; Melanoma; Melanoma, Intraocular; Merkel Cell Carcinoma; Mesothelioma, Malignant; Metastatic Squamous Neck Cancer with Occult Primary; Multiple Endocrine Neoplasia Syndrome, Childhood; Multiple Myeloma/Plasma Cell Neoplasm; Mycosis Fungoides; Myelodysplastic Syndromes; Myelogenous Leukemia, Chronic; Myeloid Leukemia, Childhood Acute; Myeloma, Multiple; Myeloproliferative Disorders, Chronic; Nasal Cavity and Paranasal Sinus Cancer; Nasopharyngeal Cancer; Nasopharyngeal Cancer, Childhood; Neuroblastoma; Non-Hodgkin's Lymphoma, Adult; Non-Hodgkin's Lymphoma, Childhood; Non-Hodgkin's Lymphoma During Pregnancy; Non-Small Cell Lung Cancer; Oral Cancer, Childhood; Oral Cavity and Lip Cancer; Oropharyngeal Cancer; Osteosarcoma/Malignant Fibrous Histiocytoma of Bone; Ovarian Cancer, Childhood; Ovarian Epithelial Cancer; Ovarian Germ Cell Tumor; Ovarian Low Malignant Potential Tumor; Pancreatic Cancer; Pancreatic Cancer, Childhood; Pancreatic Cancer, Islet Cell; Paranasal Sinus and Nasal Cavity Cancer; Parathyroid Cancer; Penile Cancer; Pheochromocytoma; Pineal and Supratentorial Primitive Neuroectodermal Tumors, Childhood; Pituitary Tumor; Plasma Cell Neoplasm/Multiple Myeloma; Pleuropulmonary Blastoma; Pregnancy and Breast Cancer; Pregnancy and Hodgkin's Lymphoma; Pregnancy and Non-Hodgkin's Lymphoma; Primary Central Nervous System Lymphoma; Primary Liver Cancer, Adult; Primary Liver Cancer, Childhood; Prostate Cancer; Rectal Cancer; Renal Cell (Kidney) Cancer; Renal Cell Cancer, Childhood; Renal Pelvis and Ureter, Transitional Cell Cancer; Retinoblastoma; Rhabdomyosarcoma, Childhood; Salivary Gland Cancer; Salivary Gland Cancer, Childhood; Sarcoma, Ewing's Family of Tumors; Sarcoma, Kaposi's; Sarcoma (Osteosarcoma)/Malignant Fibrous Histiocytoma of Bone; Sarcoma, Rhabdomyosarcoma, Childhood; Sarcoma, Soft Tissue, Adult; Sarcoma, Soft Tissue, Childhood; Sezary Syndrome; Skin Cancer; Skin Cancer, Childhood; Skin Cancer (Melanoma); Skin Carcinoma, Merkel Cell; Small Cell Lung Cancer; Small Intestine Cancer; Soft Tissue Sarcoma, Adult; Soft Tissue Sarcoma, Childhood; Squamous Neck Cancer with Occult Primary, Metastatic; Stomach (Gastric) Cancer; Stomach (Gastric) Cancer, Childhood; Supratentorial Primitive Neuroectodermal Tumors, Childhood; T-Cell Lymphoma, Cutaneous; Testicular Cancer; Thymoma, Childhood; Thymoma, Malignant; Thyroid Cancer; Thyroid Cancer, Childhood; Transitional Cell Cancer of the Renal Pelvis and Ureter; Trophoblastic Tumor, Gestational; Unknown Primary Site, Cancer of, Childhood; Unusual Cancers of Childhood; Ureter and Renal Pelvis, Transitional Cell Cancer; Urethral Cancer; Uterine Sarcoma; Vaginal Cancer; Visual Pathway and Hypothalamic Glioma, Childhood; Vulvar Cancer; Waldenström's Macroglobulinemia; and Wilms' Tumor. The composition and kits of the present invention may be useful to treat such types of cancer.

### Example 4: Autophagy promotes tumor cell survival and resistance to apopotosis

Autophagy is a lysosome-dependent degradative pathway frequently activated in tumor cells treated with chemotherapy or radiation. Whether autophagy observed in treated cancer cells represents a mechanism that allows tumor cells to survive therapy, or a mechanism to initiate a non-apoptotic form of programmed cell death remains controversial. To address this issue, the role of autophagy in a MYC-induced model of lymphoma generated from cells derived from p53ER^{TAM}/p53ER^{TAM} mice was examined. Such tumors are resistant to apoptosis due to a lack of nuclear p53. Systemic administration of tamoxifen led to p53 activation and tumor regression followed by tumor recurrence. p53 activation was associated with the rapid appearance of apoptotic cells and the induction of autophagy in surviving cells. Both shRNA-based and drug-based inhibition of autophagy enhanced the ability of either p53 activation or alkylating drug therapy to induce tumor regression and significantly delayed tumor recurrence in treated animals. These studies provide evidence that autophagy serves as a survival pathway in tumor cells treated with apoptosis activators.

A common feature of human cancers is the development of resistance to therapy induced apoptosis. Autophagy has been observed in human cancer cells subjected to chemotherapy or radiation. This study provides evidence that autophagy represents an adaptive response to therapeutic stress, and contributes to tumor cell resistance to apoptosis. Inhibition of autophagy with either chloroquine or shRNA enhanced therapy-induced apoptosis in a mouse model of lymphoma. These results provide the rationale for the use of autophagy inhibitors such as chloroquine in combination with therapies designed to induce apoptosis in human cancers.

### Introduction

Macroautophagy (referred to hereafter as autophagy) is an evolutionarily conserved process that allows cells to sequester cytoplasmic contents through the formation of double membrane vesicles (autophagosomes), and target them for degradation through the fusion of autophagosomes with lysosomes creating single membrane autolysosomes. A number of antineoplastic therapies have been observed to induce autophagy in human cancer cell lines. Whether autophagy induced by therapy contributes to therapeutic efficacy or represents a mechanism of resistance to therapy remains uncertain. Two arguments that favor autophagy as a reflection of the therapeutic efficacy of antineoplastic agents are: 1) persistent activation of autophagy can lead to programmed cell death, and 2) the autophagy regulator BECN1 (beclin) is a haploinsufficient tumor suppressor gene that induces autophagy when overexpressed. These findings suggest that stimulation of autophagy could be detrimental to cancer cells, and that therapies that inhibit autophagy would lead to enhanced tumor growth.

Accumulating evidence suggests that autophagy can also represent an adaptive strategy by which cells clear damaged organelles and survive bioenergetic stress. Autophagy, by targeting cytoplasmic proteins and organelles for lysosomal degradation, plays a role in recycling organelles and proteins that may be damaged by increased reactive oxygen species generated by the cellular stress associated with activated oncogenes and cancer therapies. Autophagy also promotes the survival of cells resistant to apoptosis when they are deprived of extracellular nutrients or growth factors.

To test the role of autophagy in tumor cell sensitivity/resistance to apoptosis a mouse model of B cell lymphoma was used. This model utilizes a p53-estrogen receptor knock-in mouse (p53ER^{TAM}/p53ER^{TAM}) which allows for the in vivo temporal dissection of the effects of p53 activation (Christophorou et al., 2005). Bone marrow cells from these mice were infected in vivo with a MYC-expressing retrovirus at high multiplicity in the flanks of recipient mice. This reproducibly gave rise to polyclonal Myc/PS3ER^{TAM} lymphomas with a B cell phenotype. These tumors can be utilized in therapeutic studies because of their ability to be adoptively transferred to the flanks of syngeneic mice. In the absence of therapy, the resulting tumors grow rapidly as they are effectively MYC positive, p53 null. Upon systemic administration of tamoxifen (TAM), the p53ER fusion protein translocates to the nucleus restoring p53 function, initiating apoptosis and tumor regression. After a period of tumor latency, 100% of animals experience tumor recurrence despite continuous TAM treatment.

In the present study pharmacological inhibition of autophagy with chloroquine (CQ) and genetic inhibition of autophagy with shRNA against the autophagy gene ATG5 were used as two independent methods to test the effect of autophagy inhibition in Myc/p53ER^{TAM} lymphomas. Acute inhibition of autophagy with CQ led to the dose-dependent inhibition of tumor cell growth in vitro and a modest inhibition of tumor growth in vivo. Tumor cells genetically selected for stable suppression of autophagy had comparable rates of growth to wild-type tumor cells and were resistant to the growth suppressive effects of CQ. Together these data suggest that inhibition of autophagy has a limited effect on the ability of healthy tumor cells to grow in vitro or in vivo. However, both CQ and/or ATG5 shRNA had a comparable ability to enhance p53-induced tumor cell death in vitro. When tumor cells were treated in vivo by either activation of p53 or systemic administration of cyclophosphamide, concomitant treatment with chloroquine significantly enhanced tumor cell death and prolonged time to tumor recurrence. This study provides evidence that tumor cells survive therapy-induced apoptosis through the process of autophagy and provides the rationale for human trials designed to test the ability of autophagy inhibitors to enhance antineoplastic therapies.

### Results

### Effects of autophagy inhibition on tumor cell growth

The pS3ER^{TAM} fusion gene consists of a transcriptionally inactive hormone-binding region of the estrogen receptor (ER^{TAM}) fused to the entire coding region of Trp53 tumor suppressor gene. In mice homozygous for knock-in alleles encoding p53ER^{TAM} (Trp^{KIIKI}), p53-dependent gene expression is induced by systemic administration of tamoxifen. To generate B cell lymphomas, bone marrow cells were harvested from several Trp53 ^{KIIKI} mice and were transduced in vivo with the LMycSN retrovirus as previously described.

To test the effect of autophagy inhibition on proliferating tumor cells in vitro, a bulk population of cells were harvested from an original Myc/pS3ER^{TAM}tumor and cultured in the presence of interleukin-7 (IL-7) and lipopolysaccharide (LPS). Chloroquine (CQ) is a lysosomotropic 4-aminoquinoline that induces death of cells dependent on autophagy for survival. CQ impaired the proliferative expansion of Myc/p53ER^{TAM} cells in vitro. The impairment of growth by CQ was dose-dependent, but low-micromolar concentrations and prolonged exposure were required to result in significant growth impairment for CQ as a single agent (IC₅₀ 3 µM) (Figure 8B).

As an independent assessment of tumor cell dependence on autophagy, the effect of genetic inhibition of autophagy using short hairpin RNA (shRNA) against the autophagy gene ATG5 was tested in vitro. The ATG5-ATG12 complex is required for the formation of autophagosomes. Short hairpin RNA against ATG5 (shATG5) or a control expression vector pKD (vector) were introduced into primary tumor cells harvested from a Myc/pOER^{TAM}B cell lymphoma. Knockdown of ATG5 was confirmed by western blot (Figure 8C). Stable knockdown of ATG5 resulted in neither impaired nor enhanced growth compared to vector cells (Figure 8D). Interestingly, CQ (5 µM) exposure resulted in impaired growth of vector tumor cells but had no effect on the growth of tumor cells expressing shATG5.

To examine the in vivo effect of CQ treatment as a single agent, cells were harvested from a Myc/p53ER^{TAM} lymphoma and were injected subcutaneously into the flanks of syngeneic mice. After tumor formation, mice were matched for tumor volumes and randomly assigned to receive daily intraperitoneal (IP) PBS, IP CQ 50 mg/kg/day or IP CQ 60 mg/kg/day (Figure 8E). These doses are near previously reported LD₅₀ of 6878 mg/kg/day. Mice treated at these doses had no observed toxicity. CQ 50 mg/kg/day or CQ 60 mg/kg/day resulted in a modest but reproducible impairment in the rate of tumor growth compared to PBS controls. However, tumor regression was not observed in any of the CQ treated animals. Daily treatment with the CQ derivative hydroxychloroquine (HCQ) at 60 mg/kg/day resulted in similar impairment in tumor growth (data not shown).

### Chloroquine enhances p53-induced apoptosis

To determine the role of autophagy after therapeutic activation of apoptosis, Myc/pS3ER^{TAM} lymphomas were generated, mice were matched for tumor volume and randomly assigned to receive either daily TAM + PBS (TAM/PBS) IP or daily TAM + CQ 60 mg/kg/day (TAM/CQ) IP. TAM treatment led to nuclear localization of the p53ER^{TAM} fusion protein (data not shown). TAM/PBS-treated tumors regressed over several days but then all tumors resumed growth despite continued TAM therapy. TAM/CQ treatment resulted in a significant delay in tumor recurrence in comparison to TAM/PBS (Figure 9A). In separate experiments daily hydroxychloroquine (HCQ) 60 mg/kg/day IP also resulted in similar enhanced regression and delayed recurrence as CQ (data not shown). In all, 81% of mice treated with TAM/CQ or TAM/HCQ compared to 8% of mice treated with TAM/PBS-treated group had complete clinical regression of their tumor in response to therapy (p<0.005).

To further understand the effect of CQ upon p53 activation, electron micrographs of lymphoma tissue during growth were obtained from mice treated with either PBS or CQ 60 mg/kg/day IP alone for 96 hours, or during tumor regression from mice treated with either TAM/PBS or TAM/CQ at 8 hours, 24 hours and 48 hours after the initiation of TAM. Low magnification micrographs (4000X) of tumors treated with PBS alone compared to tumors treated for 48 hours with TAM/PBS or TAM/CQ after initiation of treatment demonstrate widespread cell death in TAM/CQ-treated tumors (Figure 9B).

High power electron micrographs of tumors (10,000X) treated with CQ for 96 hours show an increase in the number of identifiable autophagosomes (Figure 9C). Eight hours after initiation of TAM treatment, in the presence or absence of CQ treatment, p53 activation induced morphological changes characteristic of apoptosis, including chromatin condensation, nuclear and cytoplasmic blebbing and nuclear fragmentation. At this time point no increase in autophagosomes was observed in tumor samples obtained from mice treated with either TAM/PBS or TAM/CQ in comparison to tumor samples obtained from mice treated with PBS or CQ alone. However, at 24 hours a marked increase in autophagosome accumulation in surviving tumor cells was observed in both TAM/PBS- and TAM/CQ-treated tumors. At higher magnification electron micrographs (20,000X) the characteristic double membrane structure of autophagosomes in TAM/PBS- and TAM/CQ-treated tumors was observed. Accumulation of lamellar bodies and prominent lysosomes was observed in TAM/CQ treated tumors at 24 hours (Figure 9D). In the TAM/PBS-treated animals, the number of tumor cells containing autophagosomes from TAM/PBS-treated tumors decreased by 48 hours despite continued tamoxifen treatment. In contrast, TAM/CQ-treated tumors were almost devoid of viable tumor cells by 48 hours of treatment and were primarily composed of apoptotic corpses.

The percentage of cells with visible autophagosomes per high-powered field by electron microscopy was performed to further assess therapy-induced changes in autophagosome accumulation (Figure 10). In the absence of p53 activation, an increased percentage of tumor cells with autophagosomes was observed in CQ-treated tumors compared to PBS-treated tumors (p<0.05). p53 activation with TAM increased the percentage of tumor cells with autophagosomes 30-fold at 24 hours after the initiation of TAM/PBS treatment compared to 48 hours of PBS treatment alone (p<0.0005). At 24 hours after the initiation of TAM, no significant difference in autophagosome accumulation was noted between TAM/PBS- and TAM/CQ-treated tumors (p=n.s.). At 48 hours after the initiation of TAM, surviving tumor cells with identifiable autophagosomes were present in tumors from both treatment groups.

TUNEL staining was performed on tumor specimens to assess the number of cells undergoing apoptosis in treated tumors (Figure 11 A). At eight hours after the initiation of treatment, both TAM/PBS and TAM/CQ treatments resulted in a marked increase in TUNEL-positive tumor cells compared to PBS- and CQ-treated tumors. The number of TUNEL-positive cells decreased by 48 hours in TAM/PBS-treated but not in TAM/CQ-treated tumors. Quantification of the percentage of TUNEL-positive cells per high-powered field in treated tumors (Figure 11B) found no significant difference in the percentage of TUNEL-positive cells between PBS- and CQ-treated tumors and between TAM/PBS and TAM/CQ treated tumors at 8 hours. At 24 hours, a significantly greater percentage of TUNEL-positive tumor cells was observed in TAM/CQ-treated tumors in comparison to TAM/PBS-treated tumors. This difference persisted at 48 hours when a 7-fold difference in the percent of TUNEL positive tumor cells was observed in TAM/CQ treated tumors compared to TAM/PBS-treated tumors (p<0.001). As an independent measure of tumor cell apoptosis in treated tumors, western blot analysis of cleaved caspase 3 was performed on tumor cell lysates from TAM/PBS- and TAM/CQ-treated tumors. Increased cleaved caspase 3 was observed in tumor lysates obtained at 8 hours after the initiation of either TAM/PBS or TAM/CQ. Cleaved caspase 3 was absent in TAM/PBS-treated tumor cell lysates obtained at 48 hours, but present in TAM/CQtreated tumor lysates obtained at 48 hours (data not shown).

### Inhibition of therapy-induced autophagy enhances tumor cell death

To independently assess the effects of p53 activation and CQ on tumor cell autophagy, a GFP-LC3 fusion gene was retrovirally transduced into a bulk population of cells harvested from a primary Myc/p53ER^{TAM} lymphoma and GFP+ cells were passaged in culture. LC3 is the mammalian homologue of yeast Atg8. LC3 is processed from LC3-1 to LC3-II during autophagy. LC3-II is inserted into newly formed autophagosome membranes. Expression of GFP-LC3 provides a means to track changes in autophagosome formation in living cells. The distribution of GFP-LC3 in untreated Myc/p53ER^{TAM}/GFP-LC3 cells is diffusely cytoplasmic (Figure 12A). Some punctate GFP-LC3 fluorescence was observed in tumor cells treated with CQ. Activation of p53 with 4-hydroxytamoxifen (hTAM) resulted in an increased number of punctate LC3 associated vesicles which was further enhanced by combined treated with hTAM and CQ.

To confirm that the effects of CQ observed in vivo corresponds to a direct effect on the ability of CQ to inhibit autophagy-based survival, the effects of p53 activation in the presence of CQ was compared to the effects of p53 activation in the absence of ATG5 expression. Tumor cells were induced to undergo apoptosis when p53 was activated by hTAM treatment in vitro. p53 activation with hTAM in cells with stable knockdown of ATG5 resulted in increased cell death compared to vector control cells (Figure 12B). A similar degree of increased cell death was observed during exposure of vector control cells to hTAM/CQ compared to hTAM alone (Figures 12B and 12C). Exposure of shATG5 expressing lymphoma cells with hTAM/CQ led to no additional cell death compared to either lymphoma cells expressing shATG5 treated with hTAM or vector control cells treated with hTAM/CQ (Figure 12C). This suggests that CQ's ability to enhance p53-induced cell death is dependent on the ability to inhibit autophagy.

### Chloroquine suppresses tumor recurrence after alkylating drug therapy

In the treatment of human lymphomas, alkylating agents such as cyclophosphamide serve as first-line therapies. To determine if the inhibition of autophagy could enhance the efficacy of alkylating drug therapy in tumors resistant to apoptosis, mice bearing Myc/p53ER^{TAM} lymphomas were treated with cyclophosphamide alone or in combination with CQ. Mice with Myc/p53ER^{TAM} lymphomas were treated with a single dose of cyclophosphamide 50 mg/kg IP followed by daily treatment with either PBS or daily CQ 60 mg/kg/day IP for 13 days. Cyclophosphamide with or without CQ, lead to complete tumor regression in all treated mice. The tumors of PBS-treated mice (Figure 13) recurred after an average of 5.25 ± 1.9 days, whereas a limited course of CQ treatment delayed tumor recurrence to an average of 12.5 ± 6.6 days.

### Discussion

The results described in this report provide evidence that autophagy is an adaptive mechanism that contributes to the resistance to therapy-induced apoptosis. Induction of p53 in the Mycp53ER^{TAM}tumors results in the rapid induction of tumor cell apoptosis. Cells that survive the acute induction of p53 display activation of autophagy. Inhibition of this autophagy results in enhanced apoptosis, greater tumor regression, and delayed recurrence. This is due to direct effects of autophagy inhibitors on tumor cells since autophagy inhibition by either ATG5 shRNA or CQ enhances tumor cell apoptosis and suppresses tumor cell recovery when p53 is induced in vitro. Although CQ might have multiple effects on tumor cells that could potentially explain its ability to enhance p53-induced apoptosis, the present study demonstrates that CQ requires an intact autophagic program to impair tumor cell growth and survival in the setting of an apoptotic stimulus. CQ has been shown to deacidify lysosomes leading to inhibition of the last critical step in autophagy, the aciddependent degradation of autophagosome contents, and this is likely the basis of its antineoplastic effect. The present study above also suggests that tumor cells are not absolutely dependent on autophagy for growth and survival. However, the maintenance of an autophagic response provides tumor cells with an adaptive response to survive p53 activation or alkylating drug therapy. Together, these data demonstrate that acute inhibitors of autophagy can enhance the efficacy of therapeutic strategies designed to induce tumor cell apoptosis.

Prior studies have led to conflicting views of the role of autophagy in tumor cell biology. Suppression or deficiency of autophagy genes has been shown to enhance tumor progression leading to the conclusion that rapidly growing tumors downregulate autophagy. Consistent with this, the autophagy associated tumor suppressor gene BECN1 (beclin) is monoallelically deleted in many breast cancers, leading to reduced autophagy in the tumor cells. These observations suggest autophagy may be an important mechanism to suppress tumor cell outgrowth and raise the possibility that pharmacologic suppression of autophagy might enhance tumor cell growth/survival. In contrast, recent work has suggested that autophagy plays an important role in mammalian cell biology by providing cells an adaptive mechanism to survive bioenergetic stress as a result of either growth factor or nutrient deprivation.

The present studies were undertaken because of numerous reports of autophagy being observed following cancer cell therapy. It has been suggested that the ability of radiation or chemotherapy to induce cell death in cancer cell lines that display resistance to apoptosis depends on type II programmed cell death executed by autophagy. The data presented above demonstrate the induction of autophagy in tumor cells in vivo in response to the activation of p53, a gene commonly induced by a number of antineoplastic therapies. The present studies demonstrate that p53-induced autophagy is an adaptive response that allows cancer cells to survive an apoptotic stimulus that would otherwise lead to their demise.

Based on the ability of established neoplastic cells to grow following chronic suppression of autophagy by ATG5 shRNA or CQ treatment, it appears that tumor cells are not absolutely dependent on autophagy for growth and survival. Despite this, no reports of biallelic loss of BECN1 or any other autophagy gene have been reported in human tumors. Human cancer cells lines that have monoallelic loss of BECN1 retain a wild type copy of BECN1 and express functional beclin. Based on the present results, we hypothesize that retention of a functional BECN1 gene during tumorigenesis could still allow tumor cells to the use low levels of autophagy as a response to cellular stress that would otherwise contribute to the initiation of apoptosis.

Selection for monoallelic loss of autophagy genes during tumorigenesis may be related the reported function of autophagy in eliminating damaged or excess organelles. Yeast defective in UTH1, which encodes amitochondrial protein required for effective targeting of mitochondria for autophagic degradation, are hypersensitive to certain types of oxidant injury. Therefore, chronic suppression of autophagy over a long period of time would result in the accumulation of cellular oxidants that damage DNA increasing the likelihood of cellular transformation. The role of autophagy in suppressing the accumulation of oxidative damage to cells may account for autophagy's role as a tumor suppressor pathway.

The ability of tumor cells expressing ATG5 shRNA to grow suggests that once neoplastic proliferation is established autophagy is not absolutely required for tumor cell growth and survival. However, our data suggest that there is ongoing utilization of autophagy during both in vitro and in vivo Myc/p53ER^{TAM} tumor growth, based on the accumulation of autophagic vesicles when their clearance by lysosomes is inhibited by CQ. When tumor cells are faced with cellular stress that induces apoptosis, autophagy serves to protect against cell death. Inhibition of autophagy in the setting of an apoptotic stress enhances apoptosis. Because autophagy is only necessary to maintain cell survival in times of stress, it can serve as a tumor cell survival pathway in a haplo-sufficient manner. Thus, although autophagy may prevent the accumulation of cellular oxidant stress and subsequent DNA damage when autophagy is induced in response to an apoptotic stress it serves a survival function.

Many of the existing and experimental chemotherapeutic approaches for the treatment of cancer seek to induce tumor cell apoptosis. The data presented here demonstrate that autophagy in response to either p53 activation or alkylating drug therapy contributes to the tumor cell's ability to resist apoptosis. These studies identify CQ and the related compound HCQ as effective and selective inhibitors of autophagy that can be used in vivo. The ability of CQ to enhance therapy-induced apoptosis is not absolutely dependent on p53 status since cyclophosphamide treatment of Myc/p53ER^{TAM} lymphomas was significantly enhanced by treatment with CQ.

The current data suggest that CQ may be an important adjunct to enhance the efficacy of existing chemotherapeutic strategies without potentiating toxicity. CQ cotreatment with TAM, or with the alkylating agent cyclophosphamide did not result in additional toxicity in the treated animals. This is consistent with fact that CQ has been used safely for decades in patients for malaria prophylaxis and for the treatment of rheumatoid arthritis. The chemical structure of CQ derivatives allows them to serve as weak bases that become trapped in acidic compartments. Since glycolytic tumors have been shown to be more acidic than surrounding normal tissue, CQ derivatives may preferentially accumulate in tumor tissue and display greater efficacy in the inhibition of autophagy in tumor versus normal tissue. Systemic administration of CQ at doses roughly equivalent to human doses used to treat malaria or rheumatoid arthritis was well tolerated for up to 20 days. Although CQ has been reported to have a variety of additional cellular effects in addition to its ability suppress autophagy, the ability of CQ to enhance p53-induced apoptosis was entirely dependent on its effects on autophagy and it displayed no therapeutic efficacy in tumor cells in which autophagy was chronically suppressed by ATG5 shRNA. Although ATG5 shRNA independently enhanced p53-induced cell death, no further enhancement of cell death was observed when tumor cells expressing ATG5 shRNA were treated with CQ, demonstrating that the effects of ATG5 RNA! and CQ in enhancing p53-induced apoptosis are epistatic to each other. The fact that CQ can be combined with systemic administration of cyclophosphamide with no additional toxicity also suggests that tumor cells may be more dependent on autophagy to survive such chemotherapeutic insults than nontransformed cells. Together, these data provide the rationale for testing the combination of autophagy inhibition with CQ and systemic chemotherapy and/or radiation in order to enhance the therapeutic efficacy of existing cancer therapies.

### Experimental Procedures

### Tumor generation and tissue isolation:

All experiments were performed in accordance with approved animal safety protocols. All experiments were carried out using 8-10 week old C57BL/6X129F1 mice obtained from The Jackson Laboratory (Bar Harbor, ME). Bone marrow cell harvest and production of bone marrow-derived neoplasms followed the protocol previouslydescribed. After primary tumor formation, tumor cells were harvested in ice cold PBS by passage through a 70 µM nylon mesh (BD Bioscience, Bedford, MD) and expanded in vivo by subcutaneous injection into the flanks of syngeneic mice. For tissue analysis, all animals were sacrificed individually by CO2 asphyxiation and tissue was harvested immediately. Tumors were harvested in ice cold PBS. For each tumor, sections of visually viable tumor tissue were fixed in 10% formalin for preparation of paraffin-embedded sections, glutaraldehyde for electron microscopy (see below). Tumor cell lysates were achieved through manual agitation of remaining tumor tissue in RIPA buffer.

### Drug administration and tumor measurements:

For tamoxifen (TAM) treatment, the hormone powder (Sigma, St.Louis) was dispersed, via sonication, in peanut oil (Sigma) at a concentration of 10 mg/mL. The administration of TAM was done via daily IP injections at the dose of 1 mg per mouse.

Chloroquine(CQ) (Sigma) and hydroxychloroquine (Spectrum Chemicals, Gardena, CA) were both dissolved in PBS and administered IP. For in vitro studies CQ was dissolved in PBS. 4-hydroxytamoxifen (hTAM) (Sigma) was dissolved in ethanol. Cyclophosphamide (Sigma) was dissolved in PBS. Tumors were measured on a daily basis using calipers and tumor volume was calculated using the formula: (mm³) = AxBx[A+B]/2

### Cell Culture:

For in vitro experiments, one primary MYC/pOER^{TAM} tumor was harvested in cold PBS and tumor cells were strained through a 70 µM nylon mesh (BD Bioscience, Bedford, MD) to isolate a bulk population of tumor cells. Cells were then frozen in aliquots for future experiments. All in vitro experiments were done in complete medium consisting of RPMI 1640 medium (Invitrogen, Carlsbad, CA) supplemented with 10% heat-inactivated fetal bovine serum (Gemini, Woodland, CA), 10 units/ml penicillin/streptomycin, and 2 mM L-glutamine (Invitrogen). 10 µg/ml lipopolysaccharide (Sigma, St. Louis, MO) and 0.2 ng/ml interleukin-7 (R&D, Minneapolis, MN) were added daily. For all in vitro experiments media + supplements and drugs treatments were changed daily. Cell number was assessed by using a Coulter Z2 particle analyzer or trypan blue exclusion.

### Immunoblotting:

Cultured cells were lysed in RIPA buffer. Tumor lysates were obtained by manual agitation of tumor tissue and lysis in RIPA. Lysates were standardized for protein content and resolved by SDS-PAGE on 14% NuPAGE gels (Invitrogen, St Louis, MO). Nitrocellulose blots were probed with antibodies against cleaved caspase 3 (rabbit monoclonal antibody; 1:000) (Cell Signaling, Beverly, MA); anti-actin (mouse monoclonal antibody; 1:10,000) (Sigma, St.Louis, MO); anti-ATG5 (rabbit polyclonal antibody; 1:2000) (gift from N. Mizushima).

### Electron Microscopy and quantification of autophagosomes:

Tissue obtained from tumors was immediately fixed with 2.5% glutaraldehyde/2% formaldehyde with 0.1 M sodium cacodylate and stored at 4°C until embedding. Cells were postfixed with 2% osmium tetroxide followed by an increasing gradient dehydration step using ethanol and propylene oxide. Cells were then embedded in LX-112 medium (Ladd) and sections were cut ultrathin (90 nm), placed on uncoated copper grids, and stained with 0.2% lead citrate and 1% uranyl acetate. Images were examined with a JEOL-1010 electron microscope (JEOL) at 80 W. For quantification of cells with increased autophagosome production, the percentage of cells per high-powered field (4000x) with > 3 double-membrane vesicles and intact nuclear morphology was averaged for >4 high powered fields per tumor (at least 100 cells /tumor). Data is presented as mean ± SD.

### TUNEL staining and fluorescence imaging:

TUNEL staining was performed using the In Situ Cell Death Detection Kit, TMR Red (Roche, Penzberg, Germany) on paraffin-embedded tissue harvested from tumors per manufacturer's instructions. DAPI counterstain was used to quantify cells with intact nuclei. The percentage of TUNEL positive cells was calculated by counting the number of TUNEL positive cells/DAPI positive nuclei at 100X magnification for 4 fields for each tumor sampled. For GFP-LC3 fluorescence imaging, Myc/p53ER^{TAM}/IGFP-LC3 cells (see below) were exposed to the indicated treatments and fixed with 4% paraformaldehyde for 30 minutes at room temperature, washed three times and centrifuged onto slides. DAPI counterstain was used to identify cells with intact nuclei. All fluorescence imaging was performed and digitally captured at 100X magnification on a Nikon Eclipse E800 fluorescent microscope.

### Constructs, retroviral infection and RNA interference:

Short hairpin RNA against ATG5 was generated and cloned into the pKD expression vector (constructed from pBABE-GFP) as previously described (Lum et al., 2005). MIGR1-GFP-LC3 was constructed by cloning an Xhol site 5' to the GFP-LC3 coding sequence of the pEGFP-C1/LC3 vector (generous gift of T. Yoshimori). The Xhol/EcoR/ fragment containing the entire coding region of the GFP-LC3 fusion gene was inserted into the MCS of MIGR1 to generate the MIGR1/GFP-LC3 plasmid. For production of high titer retrovirus, 293T cells were co-transfected with retroviral vector (5µg) +helper DNA (2.5µg) using Lipofectamine 2000 (Invitrogen, Carlsbad, CA). The following retroviral vectors were used: MIGR1/GFP-LC3, pKD, or pKDshATG5. Conditioned media was harvested and filtered through a 0.45-µM filter. Culture supernatants were then used to transduce MYC/p53ER^{TAM} cells. Two million MYC/p53ER^{TAM} cells were plated in 1 ml of conditioned media containing MIGR1-GFP-LC3, hairpin vector pKD, and pKDshATG5 in the presence of Hexadimethrine bromide (Polybrene; Sigma) 8 µg/ml, with fresh IL-7 and LPS added at the indicated concentrations in a 24 well plate. Culture plates were spun at 2500 rpm for 1 h at room temperature (spinfection), and then incubated at 37°C for 2 hours. Spinfection was repeated in this fashion three times. Cells were then resuspended in RPMI 10%FCS with IL-7 and LPS and expanded in culture for 3 days. Transduced cells were sorted for GFP⁺ cells (Moflo Cytomation, Fort Collins, CO) and further cultured.

## Claims

1. A pharmaceutical composition or kit comprising:
a first anti-cancer composition which is a compound that converts glycolysis dependent cancer cells to cells incapable of glycolysis and is an alkylating agent and
a second anti-cancer composition which is an autophagy inhibitor selected from the group consisting of: chloroquine, hydroxychloroquine and shRNA against the autophagy gene ATG5;
the pharmaceutical composition or kit for use in the treatment of a glycolysis dependent cancer in a human, wherein the treatment comprises administering to the individual said first anti-cancer composition and said second anti-cancer composition.

2. The pharmaceutical composition or kit for use according to claim 1 wherein the alkylating agent is a nitrosourea.

3. The pharmaceutical composition or kit for use according to claim 1 wherein the alkylating agent is selected from the group consisting of: busulfan, cisplatin, carboplatin, chlorambucil, cyclophosphamide, ifosfamide, dacarbazine (DTIC), mechlorethamine (nitrogen mustard), melphalan, carmustine (BCNU), and lomustine (CCNU).

4. The pharmaceutical composition or kit for use according to any one of claims 1 to 3 wherein the cancer is selected from the group consisting of: lung, colon, breast, prostate, pancreas, lymphoid, stomach, rectum, brain, melanoma, ovarian, testicular and bone.

5. The pharmaceutical composition or kit for use according to any one of claims 1 to 4 wherein the individual has been identified as having a glycolysis dependent cancer by performing a PETscan or by testing a sample of cancer cells obtained from the individual prior to administration of the first anti-cancer compound for glycolysis activity.

6. The pharmaceutical composition or kit for use according to claim 1, wherein the first anti-cancer agent is cyclophosphamide.

7. The use of a combination of:
an anti-cancer composition, wherein said anti-cancer composition is a compound that converts glycolysis dependent cancer cells to cells incapable of glycolysis and is an alkylating agent, and
an autophagy inhibitor selected from the group consisting of: chloroquine, hydroxychloroquine, and shRNA against the autophagy gene ATG5;
in the manufacture of a medicament for treating a glycolysis dependent cancer.

8. The use of the combination of an anti-cancer agent and an autophagy inhibitor as claimed in claim 7, wherein the alkylating agent is a nitrosourea.

9. The use of the combination of an anti-cancer agent and an autophagy inhibitor as claimed in claim 7, wherein the alkylating agent is selected from the group consisting of: busulfan, cisplatin, carboplatin, chlorambucil, cyclophosphamide, ifosfamide, dacarbazine (DTIC), mechlorethamine (nitrogen mustard), melphalan, carmustine (BCNU), and lomustine (CCNU).

10. The use of the combination of an anti-cancer agent and an autophagy inhibitor as claimed in claim 7, wherein the individual has been identified as having a glycolysis dependent cancer by performing a PETscan or by testing a sample of cancer cells obtained from the individual prior to administration of the anti-cancer compound for glycolysis activity.

11. The use of the combination of an anti-cancer agent and an autophagy inhibitor as claimed in claim 7, wherein the anti-cancer agent is cyclophosphamide.

12. The use of the combination of an anti-cancer agent and an autophagy inhibitor as claimed in any one of claims 7 to 11, wherein the cancer is selected from the group consisting of: lung, colon, breast, prostate, pancreas, lymphoid, stomach, rectum, brain, melanoma, ovarian, testicular and bone.

13. A pharmaceutical composition comprising an autophagy inhibitor selected from the group consisting of: chloroquine, hydroxychloroquine, and a shRNA against the autophagy gene ATG5 for use in the treatment of a glycolysis dependent cancer, wherein the autophagy inhibitor is for simultaneous, separate or sequential administration with an anti-cancer composition that converts glycolysis dependent cancer cells to cells incapable of glycolysis, and wherein the anti-cancer composition is an alkylating agent.

14. An autophagy inhibitor for use in the treatment of a glycolysis dependent cancer, wherein the autophagy inhibitor is selected from the group consisting of: chloroquine, hydroxychloroquine, and a shRNA against the autophagy gene ATG5 and is for simultaneous, separate or sequential administration with an anti-cancer composition that converts glycolysis dependent cancer cells to cells incapable of glycolysis, and wherein the anti-cancer composition is an alkylating agent.

15. The pharmaceutical composition for use as claimed in claim 13, or the autophagy inhibitor for use as claimed in claim 14, wherein the alkylating agent is a nitrosourea.

16. The pharmaceutical composition for use as claimed in claim 13, or the autophagy inhibitor for use as claimed in claim 14, wherein the alkylating agent is selected from the group consisting of busulfan, cisplatin, carboplatin, chlorambucil, cyclophosphamide, ifosfamide, dacarbazine (DTIC), mechlorethamine (nitrogen mustard), melphalan, carmustine (BCNU), and lomustine (CCNU).

17. The pharmaceutical composition for use as claimed in claim 13, or the autophagy inhibitor for use as claimed in claim 14, wherein the autophagy inhibitor is cyclophosphamide.

18. The pharmaceutical composition for use as claimed in any one of claims 13 or 15 to 17, or the autophagy inhibitor for use as claimed in any one of claims 14 to 17, wherein the cancer is selected from the group consisting of lung, colon, breast, prostate, pancreas, lymphoid, stomach, rectum, brain, melanoma, ovarian, testicular and bone.

## Patentansprüche

1. Pharmazeutische Zusammensetzung oder Kit, umfassend:
eine erste Antikrebs-Zusammensetzung, welche eine Verbindung ist, die von Glykolyse abhängige Krebszellen zu Zellen umwandelt, die zur Glykolyse unfähig sind, und die ein Alkylierungsmittel ist, und
eine zweite Antikrebs-Zusammensetzung, welche ein Autophagie-Inhibitor ist, der ausgewählt ist aus der Gruppe, bestehend aus: Chloroquin, Hydroxychloroquin und einer shRNA gegen das Autophagie-Gen ATG5;
wobei die pharmazeutische Zusammensetzung oder Kit zur Verwendung bei der Behandlung eines von Glykolyse abhängigen Krebses in einem Menschen ist, wobei die Behandlung das Verabreichen an das Individuum der ersten Antikrebs-Zusammensetzung und der zweiten Antikrebs-Zusammensetzung umfasst.

2. Pharmazeutische Zusammensetzung oder Kit zur Verwendung gemäß Anspruch 1, wobei das Alkylierungsmittel Nitrosoharnstoff ist.

3. Pharmazeutische Zusammensetzung oder Kit zur Verwendung gemäß Anspruch 1, wobei das Alkylierungsmittel ausgewählt ist aus der Gruppe, bestehend aus: Busulfan, Cisplatin, Carboplatin, Chlorambucil, Cyclophosphamid, Ifosfamid, Dacarbazin (DTIC), Mechlorethamin (Stickstofflost), Melphalan, Carmustin (BCNU) und Lomustin (CCNU).

4. Pharmazeutische Zusammensetzung oder Kit zur Verwendung gemäß einem der Ansprüche 1 bis 3, wobei der Krebs ausgewählt ist aus der Gruppe, bestehend aus: Lunge, Darm, Brust, Prostata, Pankreas, Lymphoid, Magen, Enddarm, Gehirn, Melanom, Eierstock, Hoden und Knochen.

5. Pharmazeutische Zusammensetzung oder Kit zur Verwendung gemäß einem der Ansprüche 1 bis 4, wobei das Individuum als einen von Glykolyse abhängigen Krebs aufweisend durch Vornehmen eines PET-Scans oder durch Testen einer Probe von Krebszellen, die von dem Individuum vor der Verabreichung der ersten Antikrebs-Verbindung erhalten ist, auf die Glykolyseaktivität identifiziert worden ist.

6. Pharmazeutische Zusammensetzung oder Kit zur Verwendung gemäß Anspruch 1, wobei das erste Antikrebsmittel Cyclophosphamid ist.

7. Verwendung einer Kombination aus:
einer Antikrebs-Zusammensetzung, wobei diese Antikrebs-Zusammensetzung eine Verbindung ist, die von Glykolyse abhängige Krebszellen zu Zellen umwandelt, die zur Glykolyse unfähig sind, und die ein Alkylierungsmittel ist, und
einem Autophagie-Inhibitor, der ausgewählt ist aus der Gruppe, bestehend aus: Chloroquin, Hydroxychloroquin und einer shRNA gegen das Autophagie-Gen ATG5;
bei der Herstellung eines Medikaments zur Behandlung eines von Glykolyse abhängigen Krebses.

8. Verwendung der Kombination aus einem Antikrebsmittel und einem Autophagie-Inhibitor wie in Anspruch 7 beansprucht, wobei das Alkylierungsmittel Nitrosoharnstoff ist.

9. Verwendung der Kombination aus einem Antikrebsmittel und einem Autophagie-Inhibitor wie in Anspruch 7 beansprucht, wobei das Alkylierungsmittel ausgewählt ist aus der Gruppe, bestehend aus: Busulfan, Cisplatin, Carboplatin, Chlorambucil, Cyclophosphamid, Ifosfamid, Dacarbazin (DTIC), Mechlorethamin (Stickstofflost), Melphalan, Carmustin (BCNU) und Lomustin (CCNU).

10. Verwendung der Kombination aus einem Antikrebsmittel und einem Autophagie-Inhibitor wie in Anspruch 7 beansprucht, wobei das Individuum als einen von Glykolyse abhängigen Krebs aufweisend durch Vornehmen eines PET-Scans oder durch Testen einer Probe von Krebszellen, die von dem Individuum vor der Verabreichung der Antikrebs-Verbindung erhalten ist, auf die Glykolyseaktivität identifiziert worden ist.

11. Verwendung der Kombination aus einem Antikrebsmittel und einem Autophagie-Inhibitor wie in Anspruch 7 beansprucht, wobei das Antikrebsmittel Cyclophosphamid ist.

12. Verwendung der Kombination aus einem Antikrebsmittel und einem Autophagie-Inhibitor wie in einem der Ansprüche 7 bis 11 beansprucht, wobei der Krebs ausgewählt ist aus der Gruppe, bestehend aus: Lunge, Darm, Brust, Prostata, Pankreas, Lymphoid, Magen, Enddarm, Gehirn, Melanom, Eierstock, Hoden und Knochen.

13. Pharmazeutische Zusammensetzung, umfassend einen Autophagie-Inhibitor, der ausgewählt ist aus der Gruppe, bestehend aus: Chloroquin, Hydroxychloroquin und einer shRNA gegen das Autophagie-Gen ATG5, zur Verwendung bei der Behandlung eines von Glykolyse abhängigen Krebses, wobei der Autophagie-Inhibitor zur gleichzeitigen, separaten oder aufeinander folgenden Verabreichung mit einer Antikrebs-Zusammensetzung ist, die von Glykolyse abhängige Krebszellen zu Zellen umwandelt, die zur Glykolyse unfähig sind, und wobei die Antikrebs-Zusammensetzung ein Alkylierungsmittel ist.

14. Autophagie-Inhibitor zur Verwendung bei der Behandlung eines von Glykolyse abhängigen Krebses, wobei der Autophagie-Inhibitor ausgewählt ist aus der Gruppe, bestehend aus: Chloroquin, Hydroxychloroquin und einer shRNA gegen das Autophagie-Gen ATG5, und zur gleichzeitigen, separaten oder aufeinander folgenden Verabreichung mit einer Antikrebs-Zusammensetzung ist, die von Glykolyse abhängige Krebszellen zu Zellen umwandelt, die zur Glykolyse unfähig sind, und wobei die Antikrebs-Zusammensetzung ein Alkylierungsmittel ist.

15. Pharmazeutische Zusammensetzung zur Verwendung wie in Anspruch 13 beansprucht, oder der Autophagie-Inhibitor zur Verwendung wie in Anspruch 14 beansprucht, wobei das Alkylierungsmittel ein Nitrosoharnstoff ist.

16. Pharmazeutische Zusammensetzung zur Verwendung wie in Anspruch 13 beansprucht, oder der Autophagie-Inhibitor zur Verwendung wie in Anspruch 14 beansprucht, wobei das Alkylierungsmittel ausgewählt ist aus der Gruppe, bestehend aus: Busulfan, Cisplatin, Carboplatin, Chlorambucil, Cyclophosphamid, Ifosfamid, Dacarbazin (DTIC), Mechlorethamin (Stickstofflost), Melphalan, Carmustin (BCNU) und Lomustin (CCNU).

17. Pharmazeutische Zusammensetzung zur Verwendung wie in Anspruch 13 beansprucht, oder der Autophagie-Inhibitor zur Verwendung wie in Anspruch 14 beansprucht, wobei der Autophagie-Inhibitor Cyclophosphamid ist.

18. Pharmazeutische Zusammensetzung zur Verwendung wie in einem der Ansprüche 13 oder 15 bis 17 beansprucht, oder der Autophagie-Inhibitor zur Verwendung wie in einem der Ansprüche 14 bis 17 beansprucht, wobei der Krebs ausgewählt ist aus der Gruppe, bestehend aus: Lunge, Darm, Brust, Prostata, Pankreas, Lymphoid, Magen, Enddarm, Gehirn, Melanom, Eierstock, Hoden und Knochen.

## Revendications

1. Composition pharmaceutique ou kit comprenant :
une première composition anti-cancer qui est un composé qui convertit les cellules cancéreuses dépendantes de la glycolyse en cellules incapables de glycolyse et qui est un agent alkylant et
une deuxième composition anti-cancer qui est un inhibiteur de l'autophagie choisi dans le groupe constitué de : chloroquine, hydroxychloroquine et ARNsh contre le gène de l'autophagie ATG5 ;
la composition pharmaceutique ou le kit pour utilisation dans le traitement d'un cancer dépendant de la glycolyse chez un être humain, le traitement comprenant l'administration à l'individu de ladite première composition anti-cancer et de ladite deuxième composition anti-cancer.

2. Composition pharmaceutique ou kit pour utilisation selon la revendication 1 dans laquelle (lequel) l'agent alkylant est une nitrosourée.

3. Composition pharmaceutique ou kit pour utilisation selon la revendication 1 dans laquelle (lequel) l'agent alkylant est choisi dans le groupe constitué de : busulfan, cisplatine, carboplatine, chlorambucil, cyclophosphamide, ifosfamide, dacarbazine (DTIC), méchloréthamine (moutarde azotée), melphalan, carmustine (BCNU) et lomustine (CCNU).

4. Composition pharmaceutique ou kit pour utilisation selon l'une quelconque des revendications 1 à 3 dans laquelle (lequel) le cancer est choisi dans le groupe constitué de : cancer du poumon, cancer du côlon, cancer du sein, cancer de la prostate, cancer du pancréas, cancer lymphoïde, cancer de l'estomac, cancer du rectum, cancer du cerveau, mélanome, cancer de l'ovaire, cancer des testicules et cancer des os.

5. Composition pharmaceutique ou kit pour utilisation selon l'une quelconque des revendications 1 à 4 dans laquelle (lequel) l'individu a été identifié comme étant atteint d'un cancer dépendant de la glycolyse en réalisant une tomographie par émission de positons ou en testant un échantillon de cellules cancéreuses obtenu auprès de l'individu avant l'administration du premier composé anti-cancer pour déterminer l'activité de la glycolyse.

6. Composition pharmaceutique ou kit pour utilisation selon la revendication 1, dans laquelle (lequel) le premier agent anti-cancer est le cyclophosphamide.

7. Utilisation d'une combinaison de :
une composition anti-cancer, ladite composition anti-cancer étant un composé qui convertit les cellules cancéreuses dépendantes de la glycolyse en cellules incapables de glycolyse et étant un agent alkylant, et
un inhibiteur de l'autophagie choisi dans le groupe constitué de : chloroquine, hydroxychloroquine et ARNsh contre le gène de l'autophagie ATG5 ;
dans la fabrication d'un médicament destiné au traitement d'un cancer dépendant de la glycolyse.

8. Utilisation de la combinaison d'un agent anti-cancer et d'un inhibiteur de l'autophagie telle que revendiquée dans la revendication 7, dans laquelle l'agent alkylant est une nitrosourée.

9. Utilisation de la combinaison d'un agent anti-cancer et d'un inhibiteur de l'autophagie telle que revendiquée dans la revendication 7, dans laquelle l'agent alkylant est choisi dans le groupe constitué de : busulfan, cisplatine, carboplatine, chlorambucil, cyclophosphamide, ifosfamide, dacarbazine (DTIC), méchloréthamine (moutarde azotée), melphalan, carmustine (BCNU) et lomustine (CCNU).

10. Utilisation de la combinaison d'un agent anti-cancer et d'un inhibiteur de l'autophagie telle que revendiquée dans la revendication 7, dans laquelle l'individu a été identifié comme étant atteint d'un cancer dépendant de la glycolyse en réalisant une tomographie par émission de positons ou en testant un échantillon de cellules cancéreuses obtenu auprès de l'individu avant l'administration du composé anti-cancer pour déterminer l'activité de la glycolyse.

11. Utilisation de la combinaison d'un agent anti-cancer et d'un inhibiteur de l'autophagie telle que revendiquée dans la revendication 7, dans laquelle l'agent anti-cancer est le cyclophosphamide.

12. Utilisation de la combinaison d'un agent anti-cancer et d'un inhibiteur de l'autophagie telle que revendiquée dans l'une quelconque des revendications 7 à 11, dans laquelle le cancer est choisi dans le groupe constitué de: cancer du poumon, cancer du côlon, cancer du sein, cancer de la prostate, cancer du pancréas, cancer lymphoïde, cancer de l'estomac, cancer du rectum, cancer du cerveau, mélanome, cancer de l'ovaire, cancer des testicules et cancer des os.

13. Composition pharmaceutique comprenant un inhibiteur de l'autophagie choisi dans le groupe constitué de : chloroquine, hydroxychloroquine et ARNsh contre le gène de l'autophagie ATG5 pour utilisation dans le traitement d'un cancer dépendant de la glycolyse, dans laquelle l'inhibiteur de l'autophagie est destiné à une administration simultanée, séparée ou séquentielle avec une composition anti-cancer qui convertit les cellules cancéreuses dépendantes de la glycolyse en cellules incapables de glycolyse, et dans laquelle la composition anti-cancer est un agent alkylant.

14. Inhibiteur de l'autophagie pour utilisation dans le traitement d'un cancer dépendant de la glycolyse, lequel inhibiteur de l'autophagie est choisi dans le groupe constitué de : chloroquine, hydroxychloroquine et ARNsh contre le gène de l'autophagie ATG5 et est destiné à une administration simultanée, séparée ou séquentielle avec une composition anti-cancer qui convertit les cellules cancéreuses dépendantes de la glycolyse en cellules incapables de glycolyse, la composition anti-cancer étant un agent alkylant.

15. Composition pharmaceutique pour utilisation telle que revendiquée dans la revendication 13, ou inhibiteur de l'autophagie pour utilisation telle que revendiquée dans la revendication 14, dans laquelle (lequel) l'agent alkylant est une nitrosourée.

16. Composition pharmaceutique pour utilisation telle que revendiquée dans la revendication 13, ou inhibiteur de l'autophagie pour utilisation telle que revendiquée dans la revendication 14, l'agent alkylant étant choisi dans le groupe constitué de busulfan, cisplatine, carboplatine, chlorambucil, cyclophosphamide, ifosfamide, dacarbazine (DTIC), méchloréthamine (moutarde azotée), melphalan, carmustine (BCNU) et lomustine (CCNU).

17. Composition pharmaceutique pour utilisation telle que revendiquée dans la revendication 13, ou inhibiteur de l'autophagie pour utilisation telle que revendiquée dans la revendication 14, l'inhibiteur de l'autophagie étant le cyclophosphamide.

18. Composition pharmaceutique pour utilisation telle que revendiquée dans l'une quelconque des revendications 13 ou 15 à 17, ou inhibiteur de l'autophagie pour utilisation telle que revendiquée dans l'une quelconque des revendications 14 à 17, le cancer étant choisi dans le groupe constitué du cancer du poumon, du cancer du côlon, du cancer du sein, du cancer de la prostate, du cancer du pancréas, du cancer lymphoïde, du cancer de l'estomac, du cancer du rectum, du cancer du cerveau, du mélanome, du cancer de l'ovaire, du cancer des testicules et du cancer des os.


### SEQUENCE LISTING

<110> The Trustees of the university of Pennsylvania
   Thompson, Craig B.
   Lum, Julian
   Bauer, Daniel
<120> REGULATION OF AUTOPHAGY AND CELL SURVIVAL
<130> P43440EP-K/NCB
<140> EP06718839.1
   <141> 2006-01-19
<150> US 60/645,419
   <151> 2005-01-19
<150> PCT/US06/01831
   <151> 2006-01-19
<160> 2
<170> PatentIn version 3.4
<210> 1
   <211> 20
   <212> DNA
   <213> Artificial Sequence
<220>
   <223> ATG5 hp2 primer
<400> 1
   ggcattatcc aattggttta 20
<210> 2
   <211> 20
   <212> DNA
   <213> Artificial Sequence
<220>
   <223> ATG5 hp7 primer
<400> 2
   gcagaaccat actatttgct 20